# EUROPEAN PATENT SPECIFICATION

(11) **EP 2 240 506 B1**
(45) Date of publication and mention of the grant of the patent: **26.12.2012**
(21) Application number: 09701124.1
(22) Date of filing: 09.01.2009
(51) Int. Cl.: C07K 5/06, C07K 5/08, C07K 5/062, C07K 5/083, A61K 38/05, A61K 38/06, A61P 35/00

(54) **INHIBITORS OF IAP**
IAP-HEMMER
INHIBITEURS DE IAP

(30) Priority: 11.01.2008 US 20682
(43) Date of publication of application: 20.10.2010
(73) Proprietor: Genentech, Inc., South San Francisco, CA 94080 (US)
(72) Inventor: NDUBAKU, Chudi, San Francisco, California 94107 (US); FLYGARE, John A., Burlingame, California 94010 (US); COHEN, Frederick, San Francisco, California 94122 (US)
(74) Representative: Wytenburg, Wilhelmus Johannes
(86) International application number: PCT/US2009/030674
(87) International publication number: WO 2009/089502

(56) References cited:
- WO-A-2006/069063
- WO-A-2007/106192
- US-A1- 2006 014 700
- BODEN C D J ET AL: "Total Synthesis of the Thiazoline-based Cyclopeptide Cyclodidemnamide" TETRAHEDRON LETTERS, ELSEVIER, AMSTERDAM, vol. 37, no. 50, 9 December 1996 (1996-12-09), pages 9111-9114, XP004070573 ISSN: 0040-4039
- HOLDER J R ET AL: "Structure-activity relationships of the melanocortin tetrapeptide Ac-His-D-Phe-Arg-Trp-NH2 at the mouse melanocortin receptors. 4. Modifications at the Trp position" JOURNAL OF MEDICINAL CHEMISTRY, AMERICAN CHEMICAL SOCIETY. WASHINGTON.; US, vol. 45, no. 26, 19 December 2002 (2002-12-19), pages 5736-5744, XP002302531 ISSN: 0022-2623
- KATALIN ÖSZ, KATALIN VÁRNAGY, HELGA SÜLI-VARGHA, DANIELE SANNA, GIOVANNI MICERA AND IMRE SÓVÁGÓ: "Transition metal complexes of bis(imidazol-2-yl) derivatives of dipeptides" DALTON TRANSACTIONS, vol. 2003, 23 April 2003 (2003-04-23), pages 2009-2016, XP002516598
- CHRISTOPHER J MOODY, JAMES C A HUNT: "Synthesis of Virenamide b, a Cytotoxic Thiazole-Containing Peptide" JOURNAL OF ORGANIC CHEMISTRY, vol. 64, 23 October 1999 (1999-10-23), pages 8715-8717, XP002524782
- NAKAMURA M ET AL: "Stereochemistry and Total Synthesis of Dolastatin E" TETRAHEDRON LETTERS, ELSEVIER, AMSTERDAM, vol. 36, no. 28, 10 July 1995 (1995-07-10), pages 5059-5062, XP004027736 ISSN: 0040-4039
- HAMADA Y ET AL: "New methods and reagents in organic synthesis. 56. Total syntheses of patellamides B and C, cytotoxic cyclic peptides from a tunicate 2. Their real structures have been determined by their syntheses" TETRAHEDRON LETTERS, ELSEVIER, AMSTERDAM, vol. 26, no. 42, 1 January 1985 (1985-01-01), pages 5159-5162, XP022720509 ISSN: 0040-4039 [retrieved on 1985-01-01]
- NORLEY M C ET AL: "Total Synthesis and Revision of Stereochemistry of Cyclodidemnamide, a Novel Cyclopeptide from the Marine Ascidian Didemnum molle" TETRAHEDRON LETTERS, ELSEVIER, AMSTERDAM, vol. 39, no. 19, 7 May 1998 (1998-05-07), pages 3087-3090, XP004115764 ISSN: 0040-4039
- CHRIS M IRELAND, AUGUSTINE R DURSO JR., ROBERT A NEWMAN, MILES P HACKER: "Antineoplastic cyclic peptides from the marine tunicate Lissoclinum patella" JOURNAL OF ORGANIC CHEMISTRY, vol. 47, no. 10, 1982, pages 1807-1811, XP002524783
- YASUMASA HAMADA, MAKOTO SHIBATA, TAKAYUKI SHIOIRI: "NEW METHODS AND REAGENTS IN ORGANIC SYNTHESIS. 58. A SYNTHESIS OF PATELLAMIDE A, A CYTOTOXIC CYCLIC PEPTIDE FROM A TUNICATE. REVISION OF ITS PROPOSED STRUCTURE" TETRAHEDRON LETTERS, vol. 26, no. 52, 1985, pages 6501-6504, XP002524784

### Sequence Listing

<110> GENENTECH, INC. NDUBAKU, CHUDI FLYGARE, JOHN A. COHEN, FREDERICK
<120> INHIBITORS OF IAP
<130> P4162R1
<140> PCTUS0930674
   <141> 2009-01-09
<150> US 61/020,682
   <151> 2008-01-11
<160> 1
<210> 1
   <211> 133
   <212> PRT
   <213> Homo sapiens
<400> 1
   Met Gly Ser Ser His His His His His His Ser Ser Gly Leu Val
    1               5                  10                  15
   Pro Arg Gly Ser His Met Leu Glu Thr Glu Glu Glu Glu Glu Glu
                   20                  25                  30
   Gly Ala Gly Ala Thr Leu Ser Arg Gly Pro Ala Phe Pro Gly Met
                   35                  40                  45
   Gly Ser Glu Glu Leu Arg Leu Ala Ser Phe Tyr Asp Trp Pro Leu
                   50                  55                  60
   Thr Ala Glu Val Pro Pro Glu Leu Leu Ala Ala Ala Gly Phe Phe
                   65                  70                  75
   His Thr Gly His Gln Asp Lys Val Arg Cys Phe Phe Cys Tyr Gly
                   80                  85                  90
   Gly Leu Gln Ser Trp Lys Arg Gly Asp Asp Pro Trp Thr Glu His
                   95                  100                 105
   Ala Lys Trp Phe Pro Gly Cys Gln Phe Leu Leu Arg Ser Lys Gly
                   110                 115                 120
   Gln Glu Tyr Ile Asn Asn Ile His Leu Thr His Ser Leu
                   125                 130

## Description

### FIELD OF THE INVENTION

The present invention relates to organic compounds useful for therapy and/or prophylaxis in a mammal, and in particular to inhibitors of IAP proteins useful for treating cancers.

### BACKGROUND OF THE INVENTION

Apoptosis or programmed cell death is a genetically and biochemically regulated mechanism that plays an important role in development and homeostasis in invertebrates as well as vertebrates. Aberrancies in apoptosis that lead to premature cell death have been linked to a variety of developmental disorders. Deficiencies in apoptosis that result in the lack of cell death have been linked to cancer and chronic viral infections.

One of the key effector molecules in apoptosis are the caspases (cysteine containing aspartate specific proteases). Caspases are strong proteases, cleaving after aspartic acid residues and once activated, digest vital cell proteins from within the cell. Since caspases are such strong proteases, tight control of this family of proteins is necessary to prevent premature cell death. In general, caspases are synthesized as largely inactive zymogens that require proteolytic processing in order to be active. This proteolytic processing is only one of the ways in which caspases are regulated. The second mechanism is through a family of proteins that bind and inhibit caspases.

A family of molecules that inhibit caspases are the Inhibitors of Apoptosis (IAP). IAPs were originally discovered in baculovirus by their functional ability to substitute for P35 protein, an anti-apoptotic gene. IAPs have been described in organisms ranging from *Drosophila* to human. Regardless of their origin, structurally, IAPs comprise one to three Baculovirus IAP repeat (BIR) domains, and most of them also possess a carboxyl-terminal RING finger motif. The BIR domain itself is a zinc binding domain of about 70 residues comprising 4 alpha-helices and 3 beta strands, with cysteine and histidine residues that coordinate the zinc ion. It is the BIR domain that is believed to cause the anti-apoptotic effect by inhibiting the caspases and thus inhibiting apoptosis. As an example, human X-chromosome linked IAP (XIAP) inhibits caspase 3, caspase 7 and the Apaf-1-cytochrome C mediated activation of caspase 9. Caspases 3 and 7 are inhibited by the BIR2 domain of XIAP, while the BIR3 domain of XIAP is responsible for the inhibition of caspase 9 activity. XIAP is expressed ubiquitously in most adult and fetal tissues, and is overexpressed in a number of tumor cell lines of the NCI 60 cell line panel. Overexpression of XIAP in tumor cells has been demonstrated to confer protection against a variety of pro-apoptotic stimuli and promotes resistance to chemotherapy. Consistent with this, a strong correlation between XIAP protein levels and survival has been demonstrated for patients with acute myelogenous leukemia. Down-regulation of XIAP expression by antisense oligonucleotides has been shown to sensitize tumor cells to death induced by a wide range of pro-apoptotic agents, both *in vitro* and *in vivo.* Smac/DIABLO-derived peptides have also been demonstrated to sensitize a number of different tumor cell lines to apoptosis induced by a variety of pro-apoptotic drugs.

Melanoma IAP (ML-IAP) is an LAP not detectable in most normal adult tissues but is strongly upregulated in melanoma. Determination of protein structure demonstrated significant homology of the ML-IAP BIR and RING finger domains to corresponding domains present in human XIAP, C-IAP1 and C-IAP2. The BIR domain of ML-IAP appears to have the most similarities to the BIR2 and BIR3 of XIAP, C-IAP1 and C-IAP2 which appear to be responsible for the inhibition of apoptosis, as determined by deletional analysis. Furthermore, it has been demonstrated that ML-IAP could inhibit chemotherapeutic agent induced apoptosis. Agents such as adriamycin and 4-tertiary butylphenol (4-TBP) were tested in a cell culture system of melanomas overexpressing ML-IAP and the chemotherapeutic agents were significantly less effective in killing the cells when compared to a normal melanocyte control. The mechanism by which ML-IAP produces an anti-apoptotic activity is in part through inhibition of caspase 3 and 9. ML-IAP did not effectively inhibit caspases 1, 2, 6, or 8.

Since apoptosis is a strictly controlled pathway with multiple interacting factors, the discovery that IAPs themselves are regulated was not unusual. In the fruit fly Drosophila, the Reaper (rpr), Head Involution Defective (hid) and GRIM proteins physically interact with and inhibit the anti-apoptotic activity of the Drosophila family of IAPs. In the mammal, the proteins SMAC/DIABLO act to block the IAPs thereby allowing apoptosis to proceed. It was shown that during normal apoptosis, SMAC is processed into an active form and is released from the mitochondria into the cytoplasm where it physically binds to IAPs and prevents the IAP from binding to a caspase. This inhibition of the IAP allows the caspase to remain active and thus proceed with apoptosis. Interestingly, sequence homology between the IAP inhibitors shows that there is a four amino acid motif in the N-terminus of the processed, active proteins. This tetrapeptide appears to bind into a hydrophobic pocket in the BIR domain and disrupts the BIR domain binding to.

### SUMMARY OF THE INVENTION

In one aspect of the present invention there is provided novel inhibitors of IAP proteins having the general formula (I) 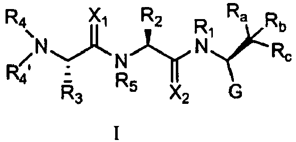 wherein
Rₐ, R_{b} and R_{c} are each independently hydroxyl, halogen, alkyl, alkoxy, alkylthio or sulfonyl; wherein said alkyl, alkoxy, alkylthio and sulfonyl groups are optionally substituted with amido, carbamoyl and aryl which are optionally substituted with hydroxyl halogen and alkoxy; or two of Rₐ, R_{b} and R_{c} together form a carbocycle or heterocycle and the other of Rₐ, R_{b} and R_{c} is H, hydroxyl, halogen, alkyl, alkoxy, alkylthio or sulfonyl; or Rₐ is H while R_{b} and R_{c} are each independently hydroxyl, halogen, alkyl, alkoxy, alkylthio or sulfonyl; wherein said alkyl, alkoxy, alkylthio and sulfonyl groups are optionally substituted with amido, carbamoyl and aryl which are optionally substituted with hydroxyl halogen and alkoxy; or two of Rₐ, R_{b} and R_{c} together form a carbocycle or heterocycle and the other of Rₐ, R_{b} and R_{c} is H, hydroxyl, halogen, alkyl, alkoxy, alkylthio or sulfonyl;
X₁ and X₂ are each independently O or S;
R₁ is H or alkyl;
R₂ is alkyl, a carbocycle, carbocyclylalkyl, a heterocycle or heterocyclylalkyl each optionally substituted with halogen, hydroxyl, oxo, thione, mercapto, carboxyl, alkyl, haloalkyl, alkoxy, alkylthio, sulfonyl, amino and nitro;
R₃ is H or alkyl optionally substituted with halogen or hydroxyl; or R₃ and R₄ together form a 3-6 heterocycle;
R₄ and R₄' are independently H, hydroxyl, amino, alkyl, carbocycle, carbocycloalkyl, carbocycloalkyloxy, carbocycloalkyloxycarbonyl, heterocycle, heterocycloalkyl, heterocycloalkyloxy or heterocycloalkyloxycarbonyl; wherein each alkyl, carbocycloalkyl, carbocycloalkyloxy, carbocycloalkyloxycarbonyl, heterocycle, heterocycloalkyl, heterocycloalkyloxy and heterocycloalkyloxycarbonyl is optionally substituted with halogen, hydroxyl, mercapto, carboxyl, alkyl, alkoxy, amino, imino and nitro; or R₄ and R₄' together form a heterocycle;
R₅ is H or alkyl;
G is 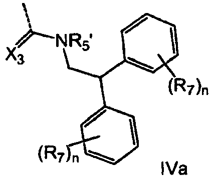 wherein
R₅' is H or alkyl;
R₇ in each occurrence is independently H, cyano, hydroxyl, mercapto, halogen, nitro, carboxyl, amidino, guanidino, alkyl, a carbocycle, a heterocycle or -U-V; wherein U is -O-, -S-, -S(O)-, S(O)₂, N(R₈)-, -C(O)-, -C(O)-NR₈-, -NR₈-C(O)-, -SO₂-NR₈-, -NR₈-SO₂-, -NR₈-C(O)-NR₈-, - NR₈-C(NH)-NR₈-, -NR₈-C(NH)-, -C(O)-O- or -O-C(O)- and V is alkyl, a carbocycle or a heterocycle; and wherein one or more CH₂ or CH groups of an alkyl is optionally replaced with -O-, -S-, -S(O)-, S(O)₂, -N(R₈)-, -C(O)-, -C(O)-NR₈-, -NR₈-C(O)-, -SO₂-NR₈-, -NR₈-SO₂-, -NR₈-C(O)-NR₈-, -C(O)-O- or -O-C(O)-; and an alkyl, carbocycle and heterocycle is optionally substituted with hydroxyl, alkoxy, acyl, halogen, mercapto, oxo, carboxyl, acyl, halo-substituted alkyl, amino, cyano nitro, amidino, guanidino an optionally substituted carbocycle or an optionally substituted heterocycle;
R₈ is H, alkyl, a carbocycle or a heterocycle wherein one or more CH₂ or CH groups of said alkyl is optionally replaced with -O-, -S-, -S(O)-, S(O)₂, -N(R₈), or -C(O)-; and said alkyl, carbocycle and heterocycle is optionally substituted with hydroxyl, alkoxy, acyl, halogen, mercapto, oxo (=O), carboxyl, acyl, halo-substituted alkyl, amino, cyano nitro, amidino, guanidino an optionally substituted carbocycle or an optionally substituted heterocycle;
X₃ is O or S;
n in each occurrence is 1 to 4;

In another aspect of the invention, there are provided compositions comprising compounds of formula I and a carrier, diluent or excipient.

Also described is a method of inducing apoptosis in a cell comprising introducing into said cell a compound of formula I.

Also described is a method of sensitizing a cell to an apoptotic signal comprising introducing into said cell a compound of formula I.

Also described is a method for inhibiting the binding of an IAP protein to a caspase protein comprising contacting said IAP protein with a compound of formula I.

The compounds of the invention are useful in a method for treating a disease or condition associated with the overexpression of an IAP protein in a mammal, comprising administering to said mammal an effective amount of a compound of formula I.

### DETAILED DESCRIPTION OF THE PREFERRED EMBODIMENTS

"Acyl," means a carbonyl containing substituent represented by the formula -C(O)-R in which R is H, alkyl, a carbocycle, a heterocycle, carbocycle-substituted alkyl or heterocycle-substituted alkyl wherein the alkyl, alkoxy, carbocycle and heterocycle are as defined herein. Acyl groups include alkanoyl (e.g. acetyl), aroyl (e.g. benzoyl), and heteroaroyl.

"Alkyl" means a branched or unbranched, saturated or unsaturated (i.e. alkenyl, alkynyl) aliphatic hydrocarbon group, having up to 12 carbon atoms unless otherwise specified. When used as part of another term, for example "alkylamino", the alkyl portion may be a saturated hydrocarbon chain, however also includes unsaturated hydrocarbon carbon chains such as "alkenylamino" and "alkynylamino. Examples of particular alkyl groups are methyl, ethyl, n-propyl, isopropyl, n-butyl, iso-butyl, sec-butyl, tert-butyl, n-pentyl, 2-methylbutyl, 2,2-dimethylpropyl, n-hexyl, 2-methylpentyl, 2,2-dimethylbutyl, n-heptyl, 3-heptyl, 2-methylhexyl, and the like. The terms "lower alkyl" "C₁-C₄ alkyl" and "alkyl of 1 to 4 carbon atoms" are synonymous and used interchangeably to mean methyl, ethyl, 1-propyl, isopropyl, cyclopropyl, 1-butyl, sec-butyl or t-butyl. Unless specified, substituted, alkyl groups may contain one, for example two, three or four substituents which may be the same or different. Examples of substituents are, unless otherwise defined, halogen, amino, hydroxyl, protected hydroxyl, mercapto, carboxy, alkoxy, nitro, cyano, amidino, guanidino, urea, sulfonyl, sulfinyl, aminosulfonyl, alkylsulfonylamino, arylsulfonylamino, aminocarbonyl, acylamino, alkoxy, acyl, acyloxy, a carbocycle, a heterocycle. Examples of the above substituted alkyl groups include, but are not limited to; cyanomethyl, nitromethyl, hydroxymethyl, trityloxymethyl, propionyloxymethyl, aminomethyl, carboxymethyl, carboxyethyl, carboxypropyl, alkyloxycarbonylmethyl, allyloxycarbonylaminomethyl, carbamoyloxymethyl, methoxymethyl, ethoxymethyl, t-butoxymethyl, acetoxymethyl, chloromethyl, bromomethyl, iodomethyl, trifluoromethyl, 6-hydroxyhexyl, 2,4-dichloro(n-butyl), 2-amino(iso-propyl), 2-carbamoyloxyethyl and the like. The alkyl group may also be substituted with a carbocycle group. Examples include cyclopropylmethyl, cyclobutylmethyl, cyclopentylmethyl, and cyclohexylmethyl groups, as well as the corresponding -ethyl, -propyl, -butyl, -pentyl, -hexyl groups, etc. Substituted alkyls include substituted methyls e.g. a methyl group substituted by the same substituents as the "substituted Cₙ-Cₘ alkyl" group. Examples of the substituted methyl group include groups such as hydroxymethyl, protected hydroxymethyl (e.g. tetrahydropyranyloxymethyl), acetoxymethyl, carbamoyloxymethyl, trifluoromethyl, chloromethyl, carboxymethyl, bromomethyl and iodomethyl.

"Amidine" means the group -C(NH)-NHR in which R is H, alkyl, a carbocycle, a heterocycle, carbocycle-substituted alkyl or heterocycle-substituted alkyl wherein the alkyl, alkoxy, carbocycle and heterocycle are as defined herein. A particular amidine is the group -NH-C(NH)-NH₂.

"Amido" means an acylamino group represented by the formula -NR-C(O)R in which each R has the meaning as defined for the respective R substituents for "amino" and "acyl" groups. Amido groups include alkanoylamino (e.g. ethanoylamino, CH₃-CO-NH-), aroylamino (e.g. benzoylamino), aralkanoylamino (e.g. phenylethanoylamino) and heterocyclecarbonylamino (e.g. piperizinylcarbonylamino.

"Amino" means primary (i.e. -NH₂), secondary (i.e. -NRH) and tertiary (i.e. -NRR) amines in which R is H, alkyl, a carbocycle, a heterocycle, carbocycle-substituted alkyl or heterocycle-substituted alkyl wherein the alkyl, alkoxy, carbocycle and heterocycle are as defined herein . Particular secondary and tertiary amines are alkylamine, dialkylamine, arylamine, diarylamine, aralkylamine and diaralkylamine wherein the alkyl is as herein defined and optionally substituted. Particular secondary and tertiary amines are methylamine, ethylamine, propylamine, isopropylamine, phenylamine, benzylamine dimethylamine, diethylamine, dipropylamine and disopropylamine.

"Amino-protecting group" as used herein refers to a derivative of the groups commonly employed to block or protect an amino group while reactions are carried out on other functional groups on the compound. Examples of such protecting groups include carbamates, amides, alkyl and aryl groups, imines, as well as many N-heteroatom derivatives which can be removed to regenerate the desired amine group. Particular amino protecting groups are Boc, Fmoc and Cbz. Further examples of these groups are found in T. W. Greene and P. G. M. Wuts, "Protective Groups in Organic Synthesis", 2nd ed., John Wiley & Sons, Inc., New York, NY, 1991, chapter 7; E. Haslam, "Protective Groups in Organic Chemistry", J. G. W. McOmie, Ed., Plenum Press, New York, NY, 1973, Chapter 5, and T.W. Greene, "Protective Groups in Organic Synthesis", John Wiley and Sons, New York, NY, 1981. The term "protected amino" refers to an amino group substituted with one of the above amino-protecting groups.

"Aryl" when used alone or as part of another term means a carbocyclic aromatic group whether or not fused having the number of carbon atoms designated or if no number is designated, up to 14 carbon atoms. Particular aryl groups are phenyl, naphthyl, biphenyl, phenanthrenyl, naphthacenyl, and the like (see e.g. Lank Handbook of Chemistry (Dean, J. A., ed) 13th ed. Table 7-2 [1985]). A particular aryl is phenyl. Substituted phenyl or substituted aryl means a phenyl group or aryl group substituted with one, two, three, four or five, for example 1-2, 1-3 or 1-4 substituents chosen, unless otherwise specified, from halogen (F, Cl, Br, I), hydroxy, protected hydroxy, cyano, nitro, alkyl (for example C₁-C₆ alkyl), alkoxy (for example C₁-C₆ alkoxy), benzyloxy, carboxy, protected carboxy, carboxymethyl, protected carboxymethyl, hydroxymethyl, protected hydroxymethyl, aminomethyl, protected aminomethyl, trifluoromethyl, alkylsulfonylamino, alkylsulfonylaminoalkyl, arylsulfonylamino, arylsulonylaminoalkyl, heterocyclylsulfonylamino, heterocyclylsulfonylaminoalkyl, heterocyclyl, aryl, or other groups specified. One or more methyne (CH) and/or methylene (CH₂) groups in these substituents may in turn be substituted with a similar group as those denoted above. Examples of the term "substituted phenyl" includes but is not limited to a mono- or di(halo)phenyl group such as 2-chlorophenyl, 2-bromophenyl, 4-chlorophenyl, 2,6-dichlorophenyl, 2,5-dichlorophenyl, 3,4-dichlorophenyl, 3-chlorophenyl, 3-bromophenyl, 4-bromophenyl, 3,4-dibromophenyl, 3-chloro-4-fluorophenyl, 2-fluorophenyl and the like; a mono- or di(hydroxy)phenyl group such as 4-hydroxyphenyl, 3-hydroxyphenyl, 2,4-dihydroxyphenyl, the protected-hydroxy derivatives thereof and the like; a nitrophenyl group such as 3- or 4-nitrophenyl; a cyanophenyl group, for example, 4-cyanophenyl; a mono- or di(lower alkyl)phenyl group such as 4-methylphenyl, 2,4-dimethylphenyl, 2-methylphenyl, 4-(iso-propyl)phenyl, 4-ethylphenyl, 3-(n-propyl)phenyl and the like; a mono or di(alkoxy)phenyl group, for example, 3,4-dimethoxyphenyl, 3-methoxy-4-benzyloxyphenyl, 3-methoxy-4-(1-chloromethyl)benzyloxy-phenyl, 3-ethoxyphenyl, 4-(isopropoxy)phenyl, 4-(t-butoxy)phenyl, 3-ethoxy-4-methoxyphenyl and the like; 3- or 4-trifluoromethylphenyl; a mono- or dicarboxyphenyl or (protected carboxy)phenyl group such 4-carboxyphenyl, ; a mono- or di(hydroxymethyl)phenyl or (protected hydroxymethyl)phenyl such as 3-(protected hydroxymethyl)phenyl or 3,4-di(hydroxymethyl)phenyl; a mono- or di(aminomethyl)phenyl or (protected aminomethyl)phenyl such as 2-(aminomethyl)phenyl or 2,4-(protected aminomethyl)phenyl; or a mono- or di(N-(methylsulfonylamino))phenyl such as 3-(N-methylsulfonylamino))phenyl. Also, the term "substituted phenyl" represents disubstituted phenyl groups where the substituents are different, for example, 3-methyl-4-hydroxyphenyl, 3-chloro-4-hydroxyphenyl, 2-methoxy-4-bromophenyl, 4-ethyl-2-hydroxyphenyl, 3-hydroxy-4-nitrophenyl, 2-hydroxy-4-chlorophenyl, and the like, as well as trisubstituted phenyl groups where the substituents are different, for example 3-methoxy-4-benzyloxy-6-methyl sulfonylamino, 3-methoxy-4-benzyloxy-6-phenyl sulfonylamino, and tetrasubstituted phenyl groups where the substituents are different such as 3-methoxy-4-benzyloxy-5-methyl-6-phenyl sulfonylamino. Particular substituted phenyl groups include the 2-chlorophenyl, 2-aminophenyl, 2-bromophenyl, 3-methoxyphenyl, 3-ethoxy-phenyl, 4-benzyloxyphenyl, 4-methoxyphenyl, 3-ethoxy-4-benzyloxyphenyl, 3,4-diethoxyphenyl, 3-methoxy-4-benzyloxyphenyl, 3-methoxy-4-(1-chloromethyl)benzyloxy-phenyl, 3-methoxy-4-(1-chloromethyl)benzyloxy -6- methyl sulfonyl aminophenyl groups. Fused aryl rings may also be substituted with any, for example 1, 2 or 3, of the substituents specified herein in the same manner as substituted alkyl groups.

"Carbamoyl" means an aminocarbonyl containing substituent represented by the formula - C(O)N(R)₂ in which R is H, hydroxyl, alkoxy, alkyl, a carbocycle, a heterocycle, carbocycle-substituted alkyl or alkoxy, or heterocycle-substituted alkyl or alkoxy wherein the alkyl, alkoxy, carbocycle and heterocycle are as herein defined. Carbamoyl groups include alkylaminocarbonyl (e.g. ethylaminocarbonyl, Et-NH-CO-), arylaminocarbonyl (e.g. phenylaminocarbonyl), aralkylaminocarbonyl (e.g. benzoylaminocarbonyl) a heterocycleaminocarbonyl (e.g. piperizinylaminocarbonyl), and in particular a heteroarylaminocarbonyl (e.g. pyridylaminocarbonyl).

"Carbocyclyl", "carbocyclylic", "carbocycle" and "carbocyclo" alone and when used as a moiety in a complex group such as a carbocycloalkyl group, refers to a mono-, bi-, or tricyclic aliphatic ring having 3 to 14 carbon atoms, for example 3 to 7 carbon atoms, which may be saturated or unsaturated, aromatic or non-aromatic. Particular saturated carbocyclic groups are cyclopropyl, cyclobutyl, cyclopentyl and cyclohexyl groups. A particular saturated carbocycle is cyclopropyl. Another particular saturated carbocycle is cyclohexyl. Particular unsaturated carbocycles are aromatic e.g. aryl groups as previously defined, for example phenyl. The terms "substituted carbocyclyl", "carbocycle" and "carbocyclo" mean these groups substituted by the same substituents as the "substituted alkyl" group.

"Carboxy-protecting group" as used herein refers to one of the ester derivatives of the carboxylic acid group commonly employed to block or protect the carboxylic acid group while reactions are carried out on other functional groups on the compound. Examples of such carboxylic acid protecting groups include 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, alkyl such as t-butyl or t-amyl, trityl, 4-methoxytrityl, 4,4'-dimethoxytrityl, 4,4',4"-trimethoxytrityl, 2-phenylprop-2-yl, trimethylsilyl, t-butyldimethylsilyl, phenacyl, 2,2,2-trichloroethyl, beta-(trimethylsilyl)ethyl, beta-(di(n-butyl)methylsilyl)ethyl, p-toluenesulfonylethyl, 4-nitrobenzylsulfonylethyl, allyl, cinnamyl, 1-(trimethylsilylmethyl)prop-1-en-3-yl, and like moieties. The species of carboxy-protecting group employed is not critical so long as the derivatized carboxylic acid is stable to the condition of subsequent reaction(s) on other positions of the molecule and can be removed at the appropriate point without disrupting the remainder of the molecule. In particular, it is important not to subject a carboxy-protected molecule to strong nucleophilic bases, such as lithium hydroxide or NaOH, or reductive conditions employing highly activated metal hydrides such as LiAlH₄. (Such harsh removal conditions are also to be avoided when removing amino-protecting groups and hydroxy-protecting groups, discussed below.) Particular carboxylic acid protecting groups are the alkyl (e.g. methyl, ethyl, t-butyl), allyl, benzyl and p-nitrobenzyl groups. Similar carboxy-protecting groups used in the cephalosporin, penicillin and peptide arts can also be used to protect a carboxy group substituents. Further examples of these groups are found in T. W. Greene and P. G. M. Wuts, "Protective Groups in Organic Synthesis", 2nd ed., John Wiley & Sons, Inc., New York, N.Y., 1991, chapter 5; E. Haslam, "Protective Groups in Organic Chemistry", J. G. W. McOmie, Ed., Plenum Press, New York, N.Y., 1973, Chapter 5, and T.W. Greene, "Protective Groups in Organic Synthesis", John Wiley and Sons, New York, NY, 1981, Chapter 5. The term "protected carboxy" refers to a carboxy group substituted with one of the above carboxy-protecting groups.

"Compound(s)" include salts and solvates (e.g. hydrates) thereof.

"Guanidine" means the group -NH-C(NH)-NHR in which R is H, alkyl, a carbocycle, a heterocycle, carbocycle-substituted alkyl or heterocycle-substituted alkyl wherein the alkyl, alkoxy, carbocycle and heterocycle are as defined herein. A particular guanidine is the group - NH-C(NH)-NH₂.

"Hydroxy-protecting group" as used herein refers to a derivative of the hydroxy group commonly employed to block or protect the hydroxy group while reactions are carried out on other functional groups on the compound. Examples of such protecting groups include tetrahydropyranyloxy, benzoyl, acetoxy, carbamoyloxy, benzyl, and silylethers (e.g. TBS, TBDPS) groups. Further examples of these groups are found in T. W. Greene and P. G. M. Wuts, "Protective Groups in Organic Synthesis", 2nd ed., John Wiley & Sons, Inc., New York, NY, 1991, chapters 2-3; E. Haslam, "Protective Groups in Organic Chemistry", J. G. W. McOmie, Ed., Plenum Press, New York, NY, 1973, Chapter 5, and T.W. Greene, "Protective Groups in Organic Synthesis", John Wiley and Sons, New York, NY, 1981. The term "protected hydroxy" refers to a hydroxy group substituted with one of the above hydroxy-protecting groups.

"Heterocyclic group", "heterocyclic", "heterocycle", "heterocyclyl", or "heterocyclo" alone and when used as a moiety in a complex group such as a heterocycloalkyl group, are used interchangeably and refer to any mono-, bi-, or tricyclic, saturated or unsaturated, aromatic (heteroaryl) or non-aromatic ring having the number of atoms designated, generally from 5 to about 14 ring atoms, where the ring atoms are carbon and at least one heteroatom (nitrogen, sulfur or oxygen), for example 1 to 4 heteroatoms. Typically, a 5-membered ring has 0 to 2 double bonds and 6- or 7-membered ring has 0 to 3 double bonds and the nitrogen or sulfur heteroatoms may optionally be oxidized (e.g. SO, SO₂), and any nitrogen heteroatom may optionally be quatemized. Particular non-aromatic heterocycles are morpholinyl (morpholino), pyrrolidinyl, oxiranyl, oxetanyl, tetrahydrofuranyl, 2,3-dihydrofuranyl, 2H-pyranyl, tetrahydropyranyl, thiiranyl, thietanyl, tetrahydrothietanyl, aziridinyl, azetidinyl, 1-methyl-2-pyrrolyl, piperazinyl and piperidinyl. A "heterocycloalkyl" group is a heterocycle group as defined above covalently bonded to an alkyl group as defined above. Particular 5-membered heterocycles containing a sulfur or oxygen atom and one to three nitrogen atoms are thiazolyl, in particular thiazol-2-yl and thiazol-2-yl N-oxide, thiadiazolyl, in particular 1,3,4-thiadiazol-5-yl and 1,2,4-thiadiazol-5-yl, oxazolyl, for example oxazol-2-yl, and oxadiazolyl, such as 1,3,4-oxadiazol-5-yl, and 1,2,4-oxadiazol-5-yl. Particular 5-membered ring heterocycles containing 2 to 4 nitrogen atoms include imidazolyl, such as imidazol-2-yl; triazolyl, such as 1,3,4-triazol-5-yl; 1,2,3-triazol-5-yl, 1,2,4-triazol-5-yl, and tetrazolyl, such as 1H-tetrazol-5-yl. Particular benzo-fused 5-membered heterocycles are benzoxazol-2-yl, benzthiazol-2-yl and benzimidazol-2-yl. Particular 6-membered heterocycles contain one to three nitrogen atoms and optionally a sulfur or oxygen atom, for example pyridyl, such as pyrid-2-yl, pyrid-3-yl, and pyrid-4-yl; pyrimidyl, such as pyrimid-2-yl and pyrimid-4-yl; triazinyl, such as 1,3,4-triazin-2-yl and 1,3,5-triazin-4-yl; pyridazinyl, in particular pyridazin-3-yl, and pyrazinyl. The pyridine N-oxides and pyridazine N-oxides and the pyridyl, pyrimid-2-yl, pyrimid-4-yl, pyridazinyl and the 1,3,4-triazin-2-yl groups, are a particular group. Substituents for "optionally substituted heterocycles", and further examples of the 5- and 6-membered ring systems discussed above can be found in U.S. Patent No. 4,278,793. In a particular embodiment, such optionally substittuted heterocycle groups are substituted with hydroxyl, alkyl, alkoxy, acyl, halogen, mercapto, oxo, carboxyl, acyl, halo-substituted alkyl, amino, cyano, nitro, amidino and guanidino.

"Heteroaryl" alone and when used as a moiety in a complex group such as a heteroaralkyl group, refers to any mono-, bi-, or tricyclic aromatic ring system having the number of atoms designated where at least one ring is a 5-, 6- or 7-membered ring containing from one to four heteroatoms selected from the group nitrogen, oxygen, and sulfur, and in a particular embodiment at least one heteroatom is nitrogen (*Lang's Handbook of Chemistry, supra*)*.* Included in the definition are any bicyclic groups where any of the above heteroaryl rings are fused to a benzene ring. Particular heteroaryls incorporate a nitrogen or oxygen heteroatom. The following ring systems are examples of the heteroaryl (whether substituted or unsubstituted) groups denoted by the term "heteroaryl": thienyl, furyl, imidazolyl, pyrazolyl, thiazolyl, isothiazolyl, oxazolyl, isoxazolyl, triazolyl, thiadiazolyl, oxadiazolyl, tetrazolyl, thiatriazolyl, oxatriazolyl, pyridyl, pyrimidyl, pyrazinyl, pyridazinyl, thiazinyl, oxazinyl, triazinyl, thiadiazinyl, oxadiazinyl, dithiazinyl, dioxazinyl, oxathiazinyl, tetrazinyl, thiatriazinyl, oxatriazinyl, dithiadiazinyl, imidazolinyl, dihydropyrimidyl, tetrahydropyrimidyl, tetrazolo[1,5-b]pyridazinyl and purinyl, as well as benzo-fused derivatives, for example benzoxazolyl, benzofuryl, benzothiazolyl, benzothiadiazolyl, benzotriazolyl, benzoimidazolyl and indolyl. A particular "heteroaryl" is:. 1,3-thiazol-2-yl, 4-(carboxymethyl)-5-methyl-1,3-thiazol-2-yl, 4-(carboxymethyl)-5-methyl-1,3-thiazol-2-yl sodium salt, 1,2,4-thiadiazol-5-yl, 3-methyl-1,2,4-thiadiazol-5-yl, 1,3,4-triazol-5-yl, 2-methyl-1,3,4-triazol-5-yl, 2-hydroxy-1,3,4-triazol-5-yl, 2-carboxy-4-methyl-1,3,4-triazol-5-yl sodium salt, 2-carboxy-4-methyl-1,3,4-triazol-5-yl, 1,3-oxazol-2-yl, 1,3,4-oxadiazol-5-yl, 2-methyl-1,3,4-oxadiazol-5-yl, 2-(hydroxymethyl)-1,3,4-oxadiazol-5-yl, 1,2,4-oxadiazol-5-yl, 1,3,4-thiadiazol-5-yl, 2-thiol-1,3,4-thiadiazol-5-yl, 2-(methylthio)-1,3,4-thiadiazol-5-yl, 2-amino-1,3,4-thiadiazol-5-yl, 1H-tetrazol-5-yl, 1-methyl-1H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1H-tetrazol-5-yl sodium salt, 1-(methylsulfonic acid)-1H-tetrazol-5-yl, 1-(methylsulfonic acid)-1H-tetrazol-5-yl sodium salt, 2-methyl-1H-tetrazol-5-yl, 1,2,3-triazol-5-yl, 1-methyl-1,2,3-triazol-5-yl, 2-methyl-1,2,3-triazol-5-yl, 4-methyl-1,2,3-triazol-5-yl, pyrid-2-yl N-oxide, 6-methoxy-2-(n-oxide)-pyridaz-3-yl, 6-hydroxypyridaz-3-yl, 1-methylpyrid-2-yl, 1-methylpyrid-4-yl, 2-hydroxypyrimid-4-yl, 1,4,5,6-tetrahydro-5,6-dioxo-4-methyl-as-triazin-3-yl, 1,4,5,6-tetrahydro-4-(formylmethyl)-5,6-dioxo-as-triazin-3-yl, 2,5-dihydro-5-oxo-6-hydroxy-astriazin-3-yl, 2,5-dihydro-5-oxo-6-hydroxy-as-triazin-3-yl sodium salt, 2,5-dihydro-5-oxo-6-hydroxy-2-methyl-astriazin-3-yl sodium salt, 2,5-dihydro-5-oxo-6-hydroxy-2-methyl-as-triazin-3-yl, 2,5-dihydro-5-oxo-6-methoxy-2-methyl-as-triazin-3-yl, 2,5-dihydro-5-oxo-as-triazin-3-yl, 2,5-dihydro-5-oxo-2-methyl-as-triazin-3-yl, 2,5-dihydro-5-oxo-2,6-dimethyl-as-triazin-3-yl, tetrazolo[1,5-b]pyridazin-6-yl and 8-aminotetrazolo[1,5-b]-pyridazin-6-yl. An alternative group of "heteroaryl" includes; 4-(carboxymethyl)-5-methyl-1,3-thiazol-2-yl, 4-(carboxymethyl)-5-methyl-1,3-thiazol-2-yl sodium salt, 1,3,4-triazol-5-yl, 2-methyl-1,3,4-triazol-5-yl, 1H-tetrazol-5-yl, 1-methyl-1H-tetrazol-5-yl, 1-(1-(dimethylamino)eth-2-yl)-1H-tetrazol-5-yl, 1-(carboxymethyl)-1H-tetrazol-5-yl, 1-(carboxymethyl)-1H-tetrazol-5-yl sodium salt, 1-(methylsulfonic acid)-1H-tetrazol-5-yl, 1-(methylsulfonic acid)-1H-tetrazol-5-yl sodium salt, 1,2,3-triazol-5-yl, 1,4,5,6-tetrahydro-5,6-dioxo-4-methyl-as-triazin-3-yl, 1,4,5,6-tetrahydro-4-(2-formylmethyl)-5,6-dioxo-as-triazin-3-yl, 2,5-dihydro-5-oxo-6-hydroxy-2-methyl-as-triazin-3-yl sodium salt, 2,5-dihydro-5-oxo-6-hydroxy-2-methyl-as-triazin-3-yl, tetrazolo[1,5-b]pyridazin-6-yl, and 8-aminotetrazolo[1,5-b]pyridazin-6-yl. Heteroaryl groups are optionally substituted as described for heterocycles.

"Inhibitor" means a compound which reduces or prevents the binding of IAP proteins to caspase proteins or which reduces or prevents the inhibition of apoptosis by an IAP protein. Alternatively, "inhibitor" means a compound which prevents the binding interaction of X-IAP with caspases or the binding interaction of ML-IAP with SMAC.

"Optionally substituted" unless otherwise specified means that a group may be unsubstituted or substituted by one or more (e.g. 0, 1, 2, 3 or 4) of the substituents listed for that group in which said substituents may be the same or different. In an embodiment an optionally substituted group has 1 substituent. In another embodiment an optionally substituted group has 2 substituents. In another embodiment an optionally substituted group has 3 substituents.

"Pharmaceutically acceptable salts" include both acid and base addition salts. "Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, carbonic acid, phosphoric acid and the like, and organic acids may be selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, salicyclic acid and the like.

"Pharmaceutically acceptable base addition salts" include those derived from inorganic bases such as sodium, potassium, lithium, ammonium, calcium, magnesium, iron, zinc, copper, manganese, aluminum salts and the like. Particularly base addition salts are the ammonium, potassium, sodium, calcium and magnesium salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particularly organic non-toxic bases are isopropylamine, diethylamine, ethanolamine, trimethamine, dicyclohexylamine, choline, and caffeine.

"Sulfonyl" means a -SO₂-R group in which R is H, alkyl, a carbocycle, a heterocycle, carbocycle-substituted alkyl or heterocycle-substituted alkyl wherein the alkyl, alkoxy, carbocycle and heterocycle are as defined herein. Particular sulfonyl groups are alkylsulfonyl (i.e. -SO₂-alkyl), for example methylsulfonyl; arylsulfonyl, for example phenylsulfonyl; aralkylsulfonyl, for example benzylsulfonyl.

The present invention provides novel compounds having the general formula (I) 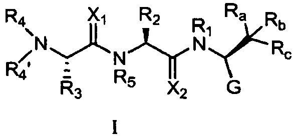 wherein G is 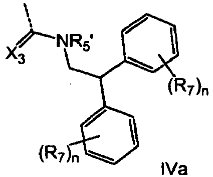 and X₁, X_{2,} X₃, R₁, R₂, R₃, R₄, R₄', R₅, R₅', R₇ and n are as described herein. In a particular embodiment G is IVa.

Rₐ, R_{b} and R_{c} are each independently hydroxyl, halogen, alkyl, alkoxy, alkylthio or sulfonyl; wherein said alkyl, alkoxy, alkylthio and sulfonyl groups are optionally substituted with amido, carbamoyl and aryl which are optionally substituted with hydroxyl halogen and alkoxy; or two of Rₐ, R_{b} and R_{c} together form a carbocycle or heterocycle and the other of Rₐ, R_{b} and R_{c} is H, hydroxyl, halogen, alkyl, alkoxy, alkylthio or sulfonyl. In a particular embodiment Rₐ, R_{b} and R_{c} are each methyl, halogen, methoxy, hydroxy, methylthio, methylsulfonyl. In a particular embodiment Rₐ, R_{b} and R_{c} are each methyl. In a particular embodiment Rₐ, R_{b} and R_{c} are each F.

In a particular embodiment two of Rₐ, R_{b} and R_{c} are methyl and the other is F. In a particular embodiment two of Rₐ, R_{b} and R_{c} are methyl and the other is hydroxyl. In a particular embodiment two of Rₐ, R_{b} and R_{c} are methyl and the other is methoxy. In a particular embodiment two of Rₐ, R_{b} and R_{c} are methyl and the other is methyl sulfonyl. In a particular embodiment two of Rₐ, R_{b} and R_{c} are methyl and the other is methylthio. In a particular embodiment two of Rₐ, R_{b} and R_{c} are methyl and the other is 4-methoxybenzylthio. In a particular embodiment two of Rₐ, R_{b} and R_{c} are methyl and the other is acetamidomethylthio. In a particular embodiment two of Rₐ, R_{b} and R_{c} together form a carbocycle or heterocycle while the other of Rₐ, R_{b} and R_{c} is H, hydroxyl, halogen, alkyl, alkoxy, alkylthio or sulfonyl. In a particular embodiment two of Rₐ, R_{b} and R_{c} form a heterocycle. In a particular embodiment two of Rₐ, R_{b} and R_{c} form a pyran. In a particular embodiment two of Rₐ, R_{b} and R_{c} form a pyran while the other is H. In a particular embodiment two of Rₐ, R_{b} and R_{c} form a pyran while the other is methyl.

Alternatively, Rₐ is H while R_{b} and R_{c} are each independently hydroxyl, halogen, alkyl, alkoxy, alkylthio or sulfonyl; wherein said alkyl, alkoxy, alkylthio and sulfonyl groups are optionally substituted with amido, carbamoyl and aryl which are optionally substituted with hydroxyl halogen and alkoxy; or two of Rₐ, R_{b} and R_{c} together form a carbocycle or heterocycle and the other of Rₐ, R_{b} and R_{c} is H, hydroxyl, halogen, alkyl, alkoxy, alkylthio or sulfonyl; provided that the compound of the invention is is other than 2-acetamido-N-(1-(1-(furan-2-yl)-2-methylpropyl-amino)-1-oxopropan-2-yl)propanamide. When Rₐ is H, R_{b} and R_{c} may be each of the particular embodiments described previously while Ra is H provided that the compound of the invention is is other than 2-acetamido-N-(1-(1-(furan-2-yl)-2-methylpropyl-amino)-1-oxopropan-2-yl)propanamide. In a particular embodiment Rₐ is H and R_{b} and R_{c} are each methyl provided that the compound of the invention is is other than 2-acetamido-N-(1-(1-(furan-2-yl)-2-methylpropyl-amino)-1-oxopropan-2-yl)propanamide.

X₁ and X₂ are each independently O or S. In a particular embodiment, X₁ and X₂ are both O. In another particular embodiment X₁ and X₂ are both S. In another particular embodiment, X₁ is S while X₂ is O. In another particular embodiment, X₁ is O while X₂ is S.

R₁ is H or alkyl. In particular embodiment R₁ is H. In particular embodiment R₁ is alkyl. In particular embodiment R₁ is methyl. In particular embodiment each of R₁, R₅ and R₅, are H. In particular embodiment R₁ is methyl while R₅ and R₅, (if present) are both H. In a particular embodiment R₁, is H, R₅ is methyl and R₅, (if present) is H.

R₂ is alkyl, a carbocycle, carbocyclylalkyl, a heterocycle or heterocyclylalkyl each optionally substituted with halogen, hydroxyl, oxo, thione, mercapto, carboxyl, alkyl, haloalkyl, alkoxy, alkylthio, acyl, hydroxyacyl, alkoxyacyl, sulfonyl, amino and nitro. In a particular embodiment R₂ is alkyl, a carbocycle, carbocyclylalkyl, a heterocycle or heterocyclylalkyl each optionally substituted with halogen, hydroxyl, oxo, mercapto, thione, carboxyl, alkyl, haloalkyl, alkoxy, alkylthio, acyl, hydroxyacyl, methoxyacyl, sulfonyl, amino and nitro. In an embodiment R₂ is alkyl, a carbocycle, carbocyclylalkyl, a heterocycle or heterocyclylalkyl each optionally substituted with halogen, hydroxyl, mercapto, carboxyl, alkyl, alkoxy, amino and nitro. In a particular embodiment R₂ is alkyl, cycloalkyl, cycloalkylalkyl, aryl, aralkyl, a heterocycle or heterocyclylalkyl. In a particular embodiment R₂ is alkyl, cycloalkyl or a heterocycle. In a particular embodiment R₂ is selected from the group consisting of t-butyl, isopropyl, cyclohexyl, tetrahydropyran-4-yl, N-methylsulfonylpiperidin-4-yl, tetrahydrothiopyran-4-yl, tetrahydrothiopyran-4-yl (in which the S is in oxidized form SO or SO₂), cyclohexan-4-one, 4-hydroxycyclohexane, 4-hydroxy-4-methylcyclohexane, 1-methyl-tetrahydropyran-4-yl, 2-hydroxyprop-2-yl, but-2-yl, thiophen-3-yl, piperidin-4-yl, N-acetylpiperidin-4-yl, N-hydroxyethylpiperidin-4-yl, N-(2-hydroxyacetyl)piperidin-4-yl, N-(2-methoxyacetyl)piperidin-4-yl, pyridin-3-yl, phenyl and 1-hydoxyeth-1-yl. In an embodiment of the invention R₂ is t-butyl, isopropyl, cyclohexyl, cyclopentyl, phenyl or tetrahydropyran-4-yl. In a particular embodiment, R₂ is phenyl. In a particular embodiment, R₂ is cyclohexyl. In another embodiment R₂ is tetrahydropyran-4-yl. In another particular embodiment, R₂ is isopropyl (i.e. the valine amino acid side chain). In another particular embodiment, R₂ is t-butyl. In a particular embodiment R₂ is oriented such that the amino acid, or amino acid analogue, which it comprises is in the L-configuration.

R₃ is H or alkyl optionally substituted with halogen or hydroxyl; or R₃ and R₄ together form a 3-6 heterocycle. In an embodiment R₃ is H or alkyl; or R₃ and R₄ together form a 3-6 heterocycle. In an embodiment R₃ is H or methyl, ethyl, propyl or isopropyl. In a particularly particular embodiment R₃ is H or methyl. In another particular embodiment R₃ is methyl. In another particular embodiment R₃ is fluoromethyl. In another particular embodiment, R₃ is ethyl. In another particular embodiment R₃ is hydroxyethyl. In a particular embodiment R₃ is fluoromethyl. In a particular embodiment R₃ is hydroxyethyl. In another embodiment R₃ is oriented such that the amino acid, or amino acid analogue, which it comprises is in the L-configuration. In a particular embodiment R₃ and R₄ together with the atoms from which they depend form a 3-6 heterocycle. In a particular embodiment R₃ and R₄ together form an azetidine ring. In a particular embodiment R₃ and R₄ together form a pyrrolidine.

R₄ and R₄' are independently H, hydroxyl, amino, alkyl, carbocycle, carbocycloalkyl, carbocycloalkyloxy, carbocycloalkyloxycarbonyl, heterocycle, heterocycloalkyl, heterocycloalkyloxy or heterocycloalkyloxycarbonyl; wherein each alkyl, carbocycloalkyl, carbocycloalkyloxy, carbocycloalkyloxycarbonyl, heterocycle, heterocycloalkyl, heterocycloalkyloxy and heterocycloalkyloxycarbonyl is optionally substituted with halogen, hydroxyl, mercapto, carboxyl, alkyl, alkoxy, amino, imino and nitro; or R₄ and R₄' together form a heterocycle. In an embodiment R₄ and R₄' are independently H, hydroxyl, amino, alkyl, aryl, aralkyl, cycloalkyl, cycloalkylalkyl, heteroaryl, or heteroarylalkyl wherein each alkyl, aryl, aralkyl, cycloalkyl, cycloalkylalkyl, heteroaryl and heteroarylalkyl is optionally substituted with halogen, hydroxyl, mercapto, carboxyl, alkyl, alkoxy, amino and nitro; or R₄ and R₄' together form a heterocycle. In a particular embodiment R₄ and R₄' together form a heterocycle, for example an azetidine ring, or a pyrrolidine ring. In a particular embodiment R₄ and R₃' are both H. In another particular embodiment R₄ is methyl and R₄' is H. In a particular embodiment one of R₄ and R₄' is hydroxyl (OH) while the other is H. In another embodiment, one of R₄ and R₄' is amino, such as NH₂, NHMe and NHEt, while the other is H. In a particular embodiment, R₄' is H and R₄ is H, alkyl, aryl, aralkyl, cycloalkyl, cycloalkylalkyl, heteroaryl or heteroarylalkyl. In a particular embodiment R₄ is a group selected from the group consisting of:

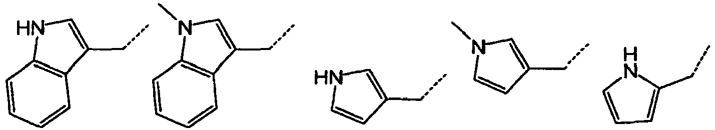

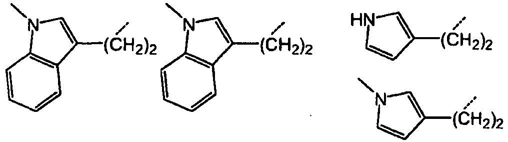

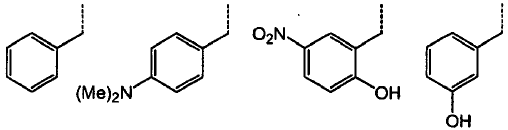

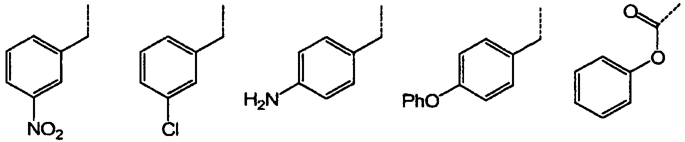

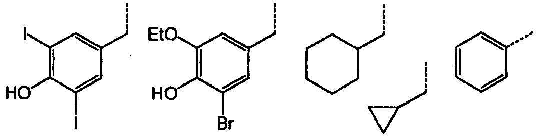

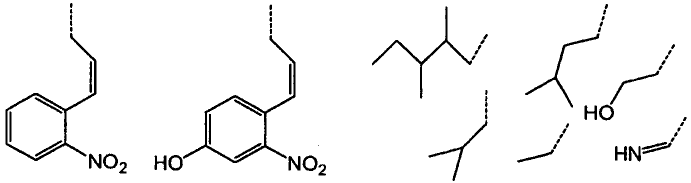

R₅ is H or alkyl. In a particular embodiment, R₅ is H or methyl. In a particular embodiment, R₅ is H. In another particular embodiment, R₅ is methyl.

R₇ in each occurrence is independently H, cyano, hydroxyl, mercapto, halogen, nitro, carboxyl, amidino, guanidino, alkyl, a carbocycle, a heterocycle or -U-V; wherein U is -O-, -S-, -S(O)-, S(O)₂, -N(R₈)-, -C(O)-, -C(O)-NR₈-, -NR₈-C(O)-, -SO₂-NR₈-, -NR₈-SO,-, -NR₈-C(O)-NR₈-, Rs-C(NH)-NR₈-, -NR₈-C(NH)-, -C(O)-O- or -O-C(O)- and V is alkyl, a carbocycle or a heterocycle; and wherein one or more CH₂ or CH groups of an alkyl is optionally replaced with -O-, -S-, -S(O)-, S(O)₂, -N(R₈)-, -C(O)-, -C(O)-NR₈-, -NR₈-C(O)-, -SO₂-NlR₈-, -NR₈-SO₂-, -NR₈-C(O)-NR₈-, -NR₈-C(NH)-NR₈-, -NR₈-C(NJ)-, -C(O)-O- or -O-C(O)-; and an alkyl, carbocycle and heterocycle is optionally substituted with hydroxyl, alkoxy, acyl, halogen, mercapto, oxo, carboxyl, acyl, halo-substituted alkyl, amino, cyano, nitro, amidino, guanidino an optionally substituted carbocycle or an optionally substituted heterocycle. Substituents of the "optionally substituted carbocycle" and "optionally substituted heterocycle" are as defined herein. In a particular embodiment such carbocycle and heterocycle groups are substituted with hydroxyl, alkyl, alkoxy, acyl, halogen, mercapto, oxo, carboxyl, acyl, halo-substituted alkyl, amino, cyano, nitro, amidino and guanidino. In an embodiment R₇ is H, halogen, alkyl, aryl, aralkyl, amino, arylamino, alkylamino, aralkylamino, alkoxy, aryloxy or aralkyloxy. In an embodiment R₇ is H, halogen, amino, hydroxyl, carboxyl, alkyl, haloalkyl or aralkyl. In a particular embodiment R₇ is halogen, for example Cl or F. In a particular embodiment R₇ is H.

R₈ is H, alkyl, a carbocycle or a heterocycle wherein one or more CH₂ or CH groups of said alkyl is optionally replaced with -O-, -S-, -S(O)-, S(O)₂, -N(R₈), or -C(O)-; and said alkyl, carbocycle and heterocycle is optionally substituted with hydroxyl, alkoxy, acyl, halogen, mercapto, oxo (=O), carboxyl, acyl, halo-substituted alkyl, amino, cyano nitro, amidino, guanidino an optionally substituted carbocycle or an optionally substituted heterocycle. Substituents of the "optionally substituted carbocycle" and "optionally substituted heterocycle" are as defined herein. In a particular embodiment such carbocycle and heterocycle groups are substituted with hydroxyl, alkyl, alkoxy, acyl, halogen, mercapto, oxo, carboxyl, acyl, halo-substituted alkyl, amino, cyano, nitro, amidino and guanidino. In a particular embodiment R₈ is H, alkyl, or acyl. In an embodiment R₈ is methyl. In another embodiment R₈ is acetyl. In a particular embodiment R₈ is H. It is understood that substitutions defined for R₇ and R₈ as well as all other variable groups herein are subject to permissible valency.

n is 1 to 4. In an embodiment n is 1. In an embodiment n is 2. In an embodiment n is 3. In an embodiment n is 4.

Compounds of the invention contain asymmetric carbon atoms. Accordingly, the compounds may exist as diastereomers, enantiomers or mixtures thereof. The syntheses of the compounds may employ racemates, diastereomers or enantiomers as starting materials or as intermediates.

Diastereomeric compounds may be separated by chromatographic or crystallization methods. Similarly, enantiomeric mixtures may be separated using the same techniques or others known in the art. Unless drawn in a particular stereochemical orientation, each of the asymmetric carbon atoms may be in the R or S configuration and both of these configurations are within the scope of the invention.

Particular compounds of formula I include the following:

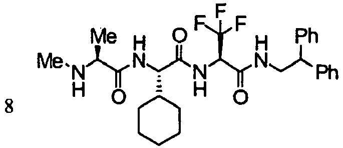

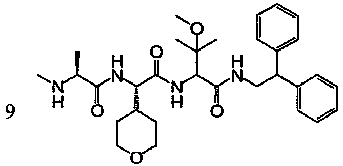

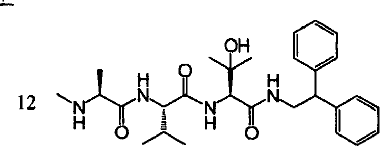

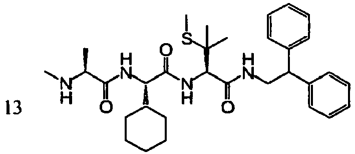

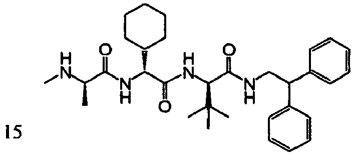

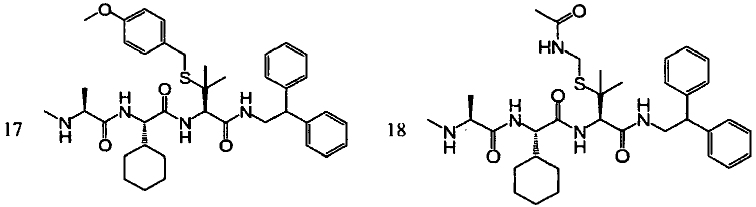

Compounds of the invention may exist in different resonance forms and that all such resonance forms are within the scope of the invention herein.

### SYNTHESIS

Compounds of the invention are prepared using standard organic synthetic techniques from commercially available starting materials and reagents. It will be appreciated that synthetic procedures employed in the preparation of compounds of the invention will depend on the particular substituents present in a compound and that various protection and deprotection steps that are standard in organic synthesis may be required but may not be illustrated in the following schemes. In a general synthetic scheme compounds of the invention may be prepared using typical peptide chemistry techniques by coupling the amino acid residue analogues with typical amide coupling procedures. For convenience, the compound of formula I can be represented by four amino acid analogue regions P1, P2, P3 and P4:

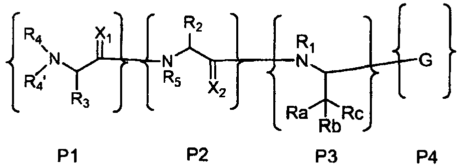

In scheme 1, amine-protected amino acid residue analogues P1 through P4 may be coupled sequentially in any order to give the final compound of formula I. For example, compouds of the invention may be prepared according to the steps shown in schemes 1a or 1b.

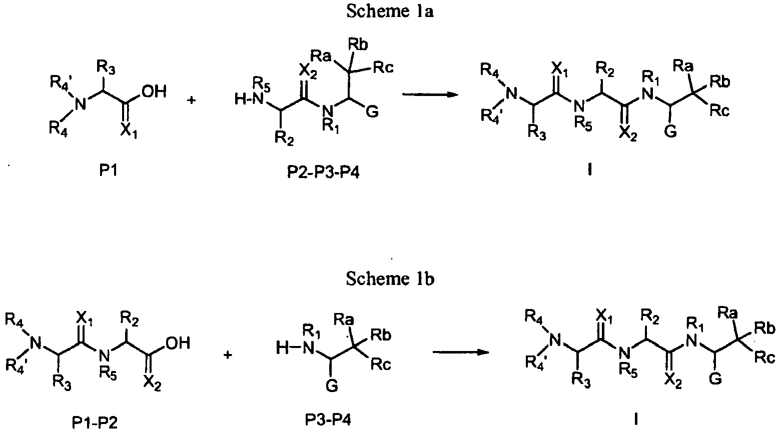

Compounds in which R₄ or R₄' are other than H may be prepared according to standard organic chemistry techniques, for example by reductive amination in which a starting amino acid residue analogue e.g. NH₂-CH(R₃)-C(O)-OH is reacted with a suitable aldehyde or ketone to give the desired R₄ and R₄' substituents as illustrated in the following scheme. The resulting R₄/R₄' substituted amino acid intermediate P1 can then be conjugated to the next amino acid intermediate P2 or the remainder of the compound (P2-P3-P4) using standard peptide coupling procedures.

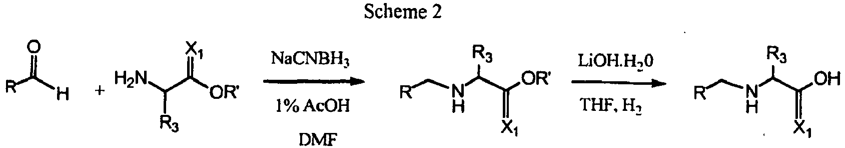

In a particular embodiment, alanine is reacted with 1-methylindole-2-carboxaldehyde and reduced with sodium cyanoborohydride dissolved in 1% HOAc/DMF to give the N-substituted alanine P1 residue which may be used in preparing compounds of the invention as shown in the following scheme.

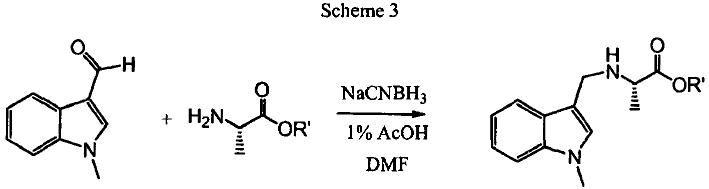

Alternatively, the reductive amination procedure to introduce R_{4/}R₄' substituents is the final step in the preparation of the compound.

When R₄ or R₄' substituents are other than H, they may also be prepared by substitution of a suitable acid intermediate incorporating a leaving group with a desired amine. For example Br-CH(R₃)-C(O)-OH is substituted with an amine R₄-NH₂ or R₄-NH-R₄' according to the following scheme.

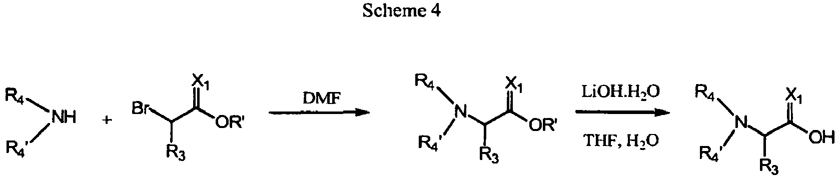

Alternatively, the substitution reaction introducing R₄ or R₄' substituents may be performed as a final step in the preparation of the compound as illustrated in the following scheme.

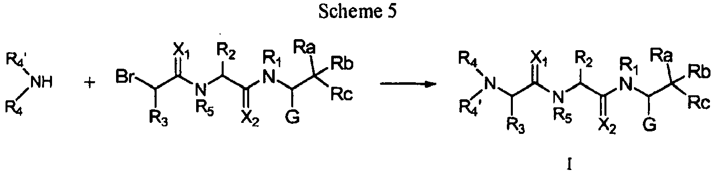

In a particular embodiment, 2-bromopropionic acid is reacted with the following amines dissolved in DMF and bubbled for until substitution is complete to form N-substituted alanine P1 residues:

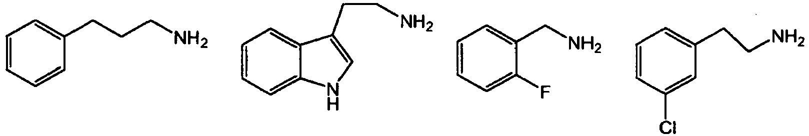

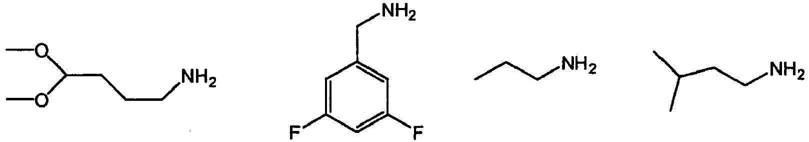

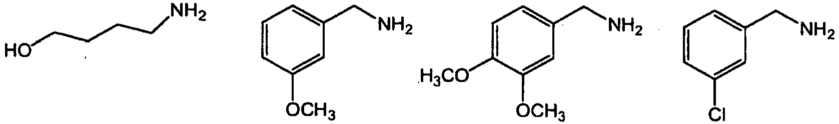

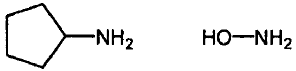

Compounds of the invention in which either X₁ or X₂ is sulfur, i.e. the compound incorporates a thioamide, may be prepared according to established organic chemistry techniques. For example, compounds in which X₂ is sulfur can be prepared starting with an Fmoc protected amino acid residue analog NH₂-CH(R₂)-COOH which is reacted with a thionating reagent such as Lawesson's Reagent or P₄S₁₀

### INDICATIONS

The compounds of the invention inhibit the binding of at least some of the IAP proteins to caspases and/or Smac. In a particular embodiment, compounds of the invention inhibit X-IAP binding to Smac. In a particular embodiment, compounds of the invention inhibit X-IAP binding interaction with caspases 3 and 7. In another particular embodiment, the compounds inhibit the binding of ML-IAP to Smac. In another particular embodiment, compounds of the invention inhibit the binding of C-IAP1 to Smac. In another particular embodiment, compounds of the invention inhibit the binding of C-IAP2 to Smac. Accordingly, the compounds of the invention are useful for inducing apoptosis in cells or sensitizing cells to apoptotic signals, in particular cancer cells. Compounds of the invention are useful for inducing apoptosis in cells that overexpress IAP proteins. Alternatively, compounds of the invention are useful for inducing apoptosis in cells in which the mitochondrial apoptotic pathway is disrupted such that release of Smac from ML-IAP proteins is inhibited, for example by up regulation of Bcl-2 or down regulation of Bax/Bak. More broadly, the compounds can be used for the treatment of all cancer types which fail to undergo apoptosis. Examples of such cancer types include neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney parenchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia lymphoma, hepatocellular carcinoma, gall bladder carcinoma, bronchial carcinoma, small cell lung carcinoma, non-small cell lung carcinoma, multiple myeloma, basalioma, teratoma, retinoblastoma, choroidea melanoma, seminoma, rhabdomyo sarcoma, craniopharyngeoma, osteosarcoma, chondrosarcoma, myosarcoma, liposarcoma, fibrosarcoma, Ewing sarcoma and plasmocytoma.

Compounds of the invention are useful for sensitizing cells to apoptotic signals. Accordingly, the compounds may be administered prior to, concomitantly with, or following administration of radiation therapy or cytostatic or antineoplastic chemotherapy. Suitable cytostatic chemotherapy compounds include, but are not limited to (i) antimetabolites, such as cytarabine, fludarabine, 5-fluoro-2'-deoxyuiridine, gemcitabine, hydroxyurea or methotrexate; (ii) DNA-fragmenting agents, such as bleomycin, (iii) DNA-crosslinking agents, such as chlorambucil, cisplatin, cyclophosphamide or nitrogen mustard; (iv) intercalating agents such as adriamycin (doxorubicin) or mitoxantrone; (v) protein synthesis inhibitors, such as L-asparaginase, cycloheximide, puromycin or diphtheria toxin; (Vi) topoisomerase I poisons, such as camptothecin or topotecan; (vii) topoisomerase II poisons, such as etoposide (VP-16) or teniposide; (viii) microtubule-directed agents, such as colcemid, colchicine, paclitaxel, vinblastine or vincristine; (ix) kinase inhibitors such as flavopiridol, staurosporin, STI571 (CPG 57148B) or UCN-01 (7-hydroxystaurosporine); (x) miscellaneous investigational agents such as thioplatin, PS-341, phenylbutyrate, ET-18- OCH₃, or farnesyl transferase inhibitors (L-739749, L-744832); polyphenols such as quercetin, resveratrol, piccatannol, epigallocatechine gallate, theaflavins, flavanols, procyanidins, betulinic acid and derivatives thereof; (xi) hormones such as glucocorticoids or fenretinide; (xii) hormone antagonists, such as tamoxifen, finasteride or LHRH antagonists. In a particular embodiment, compounds of the present invention are coadministered with a cytostatic compound selected from the group consisting of cisplatin, doxorubicin, taxol, taxotere and mitomycin C. In a particular embodiment, the cytostatic compound is doxorubicin.

Another class of active compounds which can be used in the present invention are those which are able to sensitize for or induce apoptosis by binding to death receptors ("death receptor agonists"). Such agonists of death receptors include death receptor ligands such as tumor necrosis factor a (TNF-α), tumor necrosis factor β(THF-ß, lymphotoxin-α) , LT-ß (lymphotoxin-ß), TRAIL (Apo2L, DR4 ligand), CD95 (Fas, APO-1) ligand, TRAMP (DR3, Apo-3) ligand, DR6 ligand as well as fragments and derivatives of any of said ligands. In an embodiment, the death receptor ligand is TNF-α. In a particular embodiment, the death receptor ligand is Apo2L/TRAIL. Furthermore, death receptors agonists comprise agonistic antibodies to death receptors such as anti-CD95 antibody, anti-TRAIL-R1(DR4) antibody, anti-TRAIL-R2 (DR5) antibody, anti-TRAIL-R3 antibody, anti-TRAIL-R4 antibody, anti-DR6 antibody, anti-TNF-Rl antibody and anti-TRAMP (DR3) antibody as well as fragments and derivatives of any of said antibodies.

For the purpose of sensitizing cells for apoptosis, the compounds of the present invention can be also used in combination with radiation therapy. The phrase "radiation therapy" refers to the use of electromagnetic or particulate radiation in the treatment of neoplasia. Radiation therapy is based on the principle that high-dose radiation delivered to a target area will result in the death of reproducing cells in both tumor and normal tissues. The radiation dosage regimen is generally defined in terms of radiation absorbed dose (rad), time and fractionation, and must be carefully defined by the oncologist. The amount of radiation a patient receives will depend on various consideration but the two most important considerations are the location of the tumor in relation to other critical structures or organs of the body, and the extent to which the tumor has spread. Examples of radiotherapeutic agents are provided in, but not limited to, radiation therapy and is known in the art (Hellman, Principles of Radiation Therapy, Cancer, in Principles I and Practice of Oncology, 24875 (Devita et al., 4th ed., vol 1, 1993). Recent advances in radiation therapy include three-dimensional conformal external beam radiation, intensity modulated radiation therapy (IMRT), stereotactic radiosurgery and brachytherapy (interstitial radiation therapy), the latter placing the source of radiation directly into the tumor as implanted "seeds". These newer treatment modalities deliver greater doses of radiation to the tumor, which accounts for their increased effectiveness when compared to standard external beam radiation therapy.

Ionizing radiation with beta-emitting radionuclides is considered the most useful for radiotherapeutic applications because of the moderate linear energy transfer (LET) of the ionizing particle (electron) and its intermediate range (typically several millimeters in tissue). Gamma rays deliver dosage at lower levels over much greater distances. Alpha particles represent the other extreme, they deliver very high LET dosage, but have an extremely limited range and must, therefore, be in intimate contact with the cells of the tissue to be treated. In addition, alpha emitters are generally heavy metals, which limits the possible chemistry and presents undue hazards from leakage of radionuclide from the area to be treated. Depending on the tumor to be treated all kinds of emitters are conceivable within the scope of the present invention.

Furthermore, the present invention encompasses types of non-ionizing radiation like e.g. ultraviolet (UV) radiation, high energy visible light, microwave radiation (hyperthermia therapy), infrared (IR) radiation and lasers. In a particular embodiment of the present invention UV radiation is applied.

The invention also includes pharmaceutical compositions or medicaments containing the compounds of the invention and a therapeutically inert carrier, diluent or excipient, as well as methods of using the compounds of the invention to prepare such compositions and medicaments. Typically, the compounds of formula I used in the methods of the invention are formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but may range anywhere from about 3 to about 8. Formulation in an acetate buffer at pH 5 is a suitable embodiment. In an embodiment, the inhibitory compound for use herein is sterile. The compound ordinarily will be stored as a solid composition, although lyophilized formulations or aqueous solutions are acceptable.

The composition of the invention will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to inhibit IAP interaction with caspases, induce apoptosis or sensitize a malignant cell to an apoptotic signal. Such amount is may be below the amount that is toxic to normal cells, or the mammal as a whole.

Generally, the initial pharmaceutically effective amount of the compound of the invention administered parenterally per dose will be in the range of about 0.01-100 mg/kg, for example about 0. 1to 20 mg/kg of patient body weight per day, with the typical initial range of compound used being 0.3 to 15 mg/kg/day. Oral unit dosage forms, such as tablets and capsules, may contain from about 25 to about 1000 mg of the compound of the invention.

The compound of the invention may be administered by any suitable means, including oral, topical, transdermal, parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. An example of a suitable oral dosage form is a tablet containing about 25mg, 50mg, 100mg, 250mg, or 500mg of the compound of the invention compounded with about 90-30 mg anhydrous lactose, about 5-40 mg sodium croscarmellose, about 5-30mg polyvinylpyrrolidone (PVP) K30, and about 1-10 mg magnesium stearate. The powdered ingredients are first mixed together and then mixed with a solution of the PVP. The resulting composition can be dried, granulated, mixed with the magnesium stearate and compressed to tablet form using conventional equipment. An aerosol formulation can be prepared by dissolving the compound, for example 5-400 mg, of the invention in a suitable buffer solution, e.g. a phosphate buffer, adding a tonicifier, e.g. a salt such sodium chloride, if desired. The solution is typically filtered, e.g. using a 0.2 micron filter, to remove impurities and contaminants.

### EXAMPLES

The invention will be more fully understood by reference to the following examples. Reagents and solvents were obtained from commercial sources and used as received.

Abbreviations used herein are as follows:
AcOH: acetic acid;
ACN: acetonitrile;
Chg: cyclohexylglycine;
DCM: dichloromethane
DIC: N,N'-diisopropylcarbodiimide
DIPEA: diisopropylethylamine;
DMAP: 4- dimethylaminopyridine;
DME: 1,2-dimethoxyethane;
DMF: dimethylfonnamide;
DMSO: dimethylsulfoxide
EDC: 1-ethyl-3-(3-dimethylaminopropyl)carbodiimide;
EEDQ: 2-ethoxy-1-ethoxycarbonyl-1,2-dihydroquinoline;
EtOAc: ethylacetate
EtOH: ethanol;
LCMS: liquid chromatography mass spectrometry;
HATU: O-(7-Azobenzotriazol-1-yl)-1,1,3,3-tetramethyluronium hexafluorophosphate;
HOAt: 1-hydroxy-7-azabenzotriazole
HOBt: N-hydroxybenzotriazole
HBTU: 2-(1H-Benzotriazol-1-yl)-1,1,3,3-Tetramethyl-uronium Hexafluorophosphate;
HPLC: high performance liquid chromatography;
MeOH: methanol;
NBS: N-bromosuccinamide;
PyAOP: 7-azabenzotriazol-1-yloxy-tris-(pyrrolidino)phosphonium hexafluorophosphate;
TASF: tris(dimethylamino)sulfonium difluotrimethylsilicate;
TEA: triethylamine;
TFA: trifluoroacetic acid;
THF: tetrahydrofuran;

### Example 1 2-[tert-Butoxycarbonyl-(1H-pyrrol-2-ylmethyl)-amino]-propionic acid

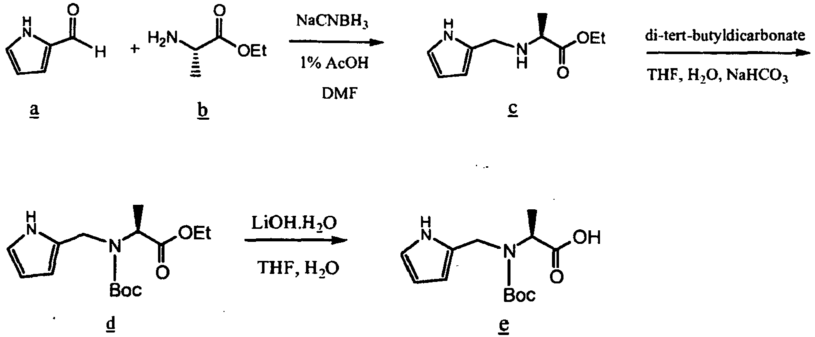

Alanine ethyl ester b (5g, 32.5mmol), pyrrole-2-carboxaldehyde a (3.1g, 32.5mmol), sodium cyanoborohydride (2.04g, 32.5mmol) and AcOH (1%) were mixed in DMF and stirred overnight. The reaction was quenched with H₂O, and DMF was evaporated. The mixture was diluted with EtOAc, washed by 0.1N NaOH, dried and concentrated to yield product c 2.5g. The resulting ester c (2.5g, 12.8mmol), di-tert-butyldicarbonate (3.06g, 14mmol) were mixed in THF, H₂O with NaHCO₃ and stirred overnight. THF was evaporated, and the mixture was diluted with EtOAc, washed by 1N NaOH, sat. NH₄Cl and brine. After dried, the mixture was concentrated to yield the Boc-protected ester d 3:3g. The Boc-protected ester d (1.67g, 5.6mol), lithium hydroxide mono hydrate (284mg, 6.77mmol) were mixed in THF and H₂O at 0°C. THF was vacuumed off, and the solution was acidified by dilute H₂SO₄, extracted by EtOAc twice. Organic layers were combined, dried and evaporated giving product 2-[tert-butoxycarbonyl-(1H-pyrrol-2-ylmethyl)-amino]-propionic acid e.

### Example 2 tetrahydropyranylglycine

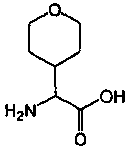

Tetrahydropyranylglycine was purchased from NovaBiochem, or synthesized according to the literature: Ghosh, A. K.; Thompson, W. J.; holloway, M. K.; McKee, S. P.; Duong, T. T.; Lee, H. Y.; Munson, P. M.; Smith, A. M.; Wai, J. M; Darke, P. L.; Zugay, J. A.; Emini, E. A.; Schleife, W. A.; Huff, J. R.; Anderson, P. S. J. Med. Chem, 1993, 36, 2300-2310.

### Example 3 piperidinylglycine

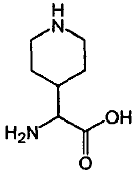

Piperidinylglycine was synthesized according to the procedures described by Shieh et al. (Tetrahedron: Asymmetry, 2001, 12, 2421-2425.

### Example 4 4,4-difluorocyclohexylglycine

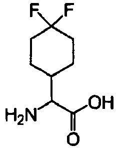

4,4-difluorocyclohexylglycine was made according to the procedures described in patent application US 20030216325.

### Example 5 Boc (S)-2-amino-2-(4-hydroxycyclohexyl)acetic acid

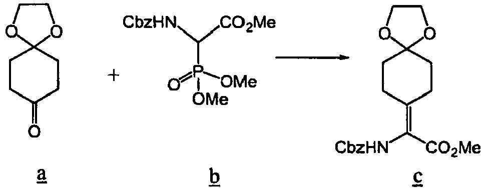

Following the procedure described by Sheih et al. (Tetrahedron: Asymmetry, 2001, 12, 2421-2425), a solution of ketone a (8.4 g) and EtOAc (30 mL) was added to a solution of *N*-Cbz-phosphonoglycine methyl ester b, TMG (4.5 mL) and EtOAc (30 mL). The solution was maintained at rt for 48h, then washed with 1N HCl (3x50 mL), brine (1x50 mL) dried (Na₂SO₄), filtered, and concentrated. The residue was adsorbed onto Celite, and purified by chromatography, then further purified by re-crystalization from EtOAc/hexanes to afford 5.2 g of product c.

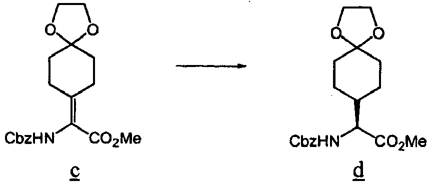

Following the procedure described by Sheih, (Tetrahedron: Asymmetry, 2001, 12, 2421-2425), a solution of eneamide c (5.0 g), (S,S)-Me-BPE-Rh(I) (1.5g, Strem Chemicals, Newburyport, MA), and MeOH (100 mL) was shaken virgorously under 70psi of H₂ for 48h. The solvent was removed under reduced pressure. The residue was taken up in EtOAc, and filtered through SiO₂ with more EtOAc. The solvent was removed under reduced pressure to afford 4.0g of product d as a colorless solid.

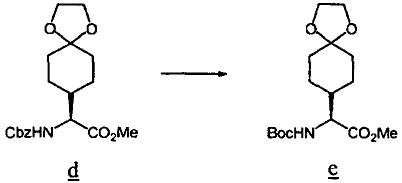

A mixture of Cbz-carbamate d, (4.0g) Boc₂O, (2.9g), 20% Pd(OH)₂•C (1.0g) and MeOH (30 mL) was maintained under an atmosphear of H₂ for 6h. The mixture was filtered through Celite with MeOH. The solvent was removed under reduced pressure to afford 4.5 g of residue e, which was taken on directly.

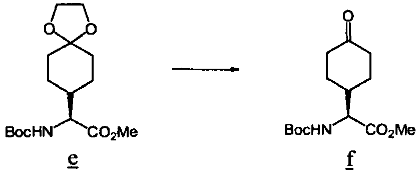

The residue e from above was dissolved in H₂O (10 mL), AcOH (30 mL), THF (5 mL), and dichloroacetic acid (3 mL) and maintained at rt overnight. Water (5 mL) was added and the solution and maintaned until hyrolysis was complete, as monitored by HPLC-MS. Solid Na₂CO₃ was added cautiously until gas evolution ceased, the mixture was diluted with aq NaHCO₃, and extracted with 10%EtOAc/DCM. The combined organic phases were washed once with brine, dried (Na₂SO₄), filtered, and concentrated. The residue was purified by chromatography to afford 2.9g of product f.

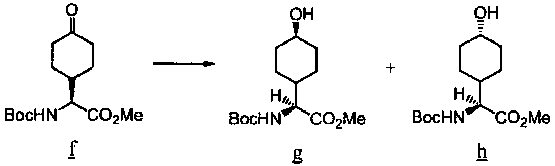

A mixture of ketone f (1.5g) MeOH (50 ml) was treated with NaBH4 (290 mg) at 0 °C for 20 min. The mixture was acidifed to ∼pH1 with 10%aq citric acid and the MeOH was removed under reduced pressure. The residue was diluted with water and extraced with 20%EtOAc/DCM. The combined organic phases were washed once with brine, dried (Na₂SO₄), filtered, and concentrated. The residue was purified by chromatography to afford 1.17g of product g and 0.23g of product h.

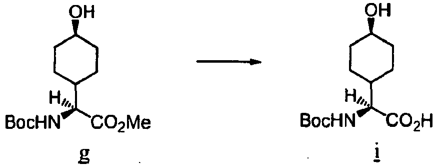

A mixture of ester g (1.17g) LiOH•H20 (160mg), THF (3 mL) and water (4.5 mL) was stirred vigorously at rt overnight. The mixture was diluted with brine and exaustivly extraced with EtOAc. The combined organic phases were washed once with brine, dried (Na₂•SO₄), filtered, and concentrated to afford acid i (525mg).

### Example 6 N-Boc-N-cyclopropylmethyl-L-alanine

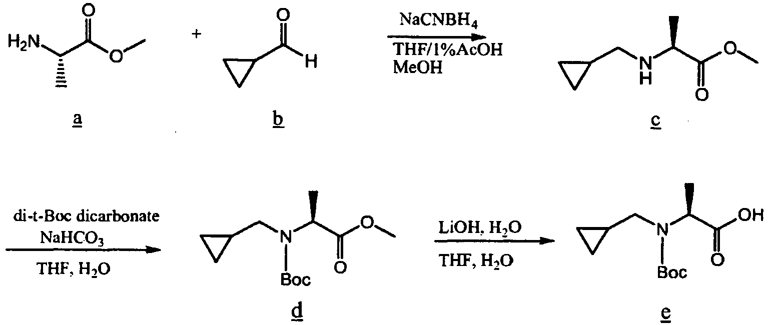

L-alanine methyl ester hydrochloride a (5g, 35.8mmol) and cyclopropanecarboxaldehyde b (2.67ml, 35.8mmol) were suspended in 50ml THF w/1% AcOH. Addition of 5ml of CH₃OH made the cloudy solution turned to clear. NaCNBH₄ (2.25g, 35.8mmol) was added and the reaction mixture stirred overnight. The reaction was quenched by addition of 1N aq. NaOH, extracted by EtOAc twice, organic layers were dried over Na₃SO₄ and concentrated to dryness. The crude material was purified by chromatography using 30% EtOAc/hexane (stained by ninhydrin) to obtain the compound c (1g, 18%). The compound c (1g, 6.37mmol) and di-t-bocdicarbonate (2.1g, 9.55mmol) were diluted in THF (20ml) and H₂O (20ml), NaHCO₃ (1.3g, 15.9mmol) was added. The reaction mixture stirred overnight for completion. THF was removed under reduced pressure, and the aqueous layer was extracted by EtOAc 3 times. Combined organic layers were washed by 1N NaOH, sat, NH₄Cl followed by brine, the concentrated to dryness. The Boc-protected compound d (1.39g, 5.40mmol) was stirred with LiOH.H₂O (1.14g, 27mmol) in THF (20ml) and H₂O (20ml) overnight at room temperature. THF was stripped off, and the aqueous layer was adjusted to pH=4 by adding 10% citric acid, then extracted by EtOAc 3 times. Combined organic layers were washed by brine and concentrated. The crude was purified by reverse phase C-18 column eluted by 0%-50% acetonitrile/H₂O to give pure compound e as a white solid (794mg).

### Example 7 N-Boc-N-methyl-L-alanine-L-cyclohexylglycine

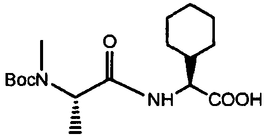

A solution of Fmoc-L-cyclohexylglycine (3.6 g, 9.6 mmol) dissolved in DCM (50 mL) and DIPEA (5.6 mL, 32 mmol) was added to 2-chlorotrityl chloride resin (5 g, 8 mmol) and gently agitated for 3 hours at room temperature. The resin was washed with DCM 4 times, DCM/MeOH/DIPEA (17:2:1) 3 times, DCM 3 times, and 2 times dimethylacetamide (DMA). The Fmoc group was removed by treating the resin with 20% piperidine/DMA (50 mL) for 15 minutes. The resin was washed with DMA 6 times. A solution of Boc-N-methylalanine (3.3 g, 16 mmol), HBTU (6.1 g, 16 mmol), and DIPEA (5.6 mL, 32 mmol) and DMA/DCM (1:1, 50 mL) was added to the resin and gently agitated for 2 hours at room temperature. The resin was washed with DMA 5 times, DCM 2 times, and dried under reduced pressure. The dipeptide was cleaved from the resin by gentle agitation with HOAc/TFE/DCM (1:1:3, 100 mL) for 2 hours at room temperature. The resin was removed by filtration and the solution concentrated. Residual AcOH was removed by azeotroping with hexanes (15 times volume). The solid residue was purified by reverse-phase HPLC (C₁₈, MeCN-H₂O, 0.1%TFA) and the solvents removed by lyophylization to provide 1.2 g (43%) of dipeptide N-Boc-N-methyl-L-alanine-L-cyclohexylglycine as a white powder.

### Example 8 N-Boc-N-methyl-L-alanine-L-dehydropyranylglycine

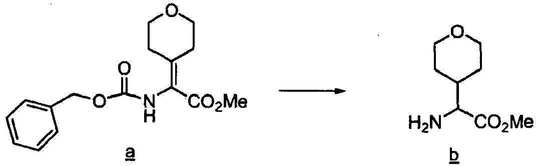

A mixture of N-Cbz-dehydropytanylglycine methyl ester a (Burk, M. J.; Gross, M. F.; Martinez, J. P. J. Am Chem. Soc. 1995, 117, 9375, and references therein) (5.2 g, 17 mmol), 5% Pd·C (500 mg), MeOH (75 mL) and THF (25 mL) was maintained under an atmosphere of H₂ for 24 h. The mixture was filtered through Celite and the Celite washed with MeOH, and concentrated under reduced pressure to afford a quantitative yield of amine b as a colorless oil, which was carried on directly.

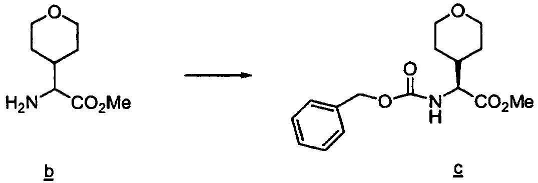

The amine b prepared above was combined with CH₂Cl₂ (40 mL), saturated aqueous NaHCO₃ (40 mL) and cooled to 0 °C. Benzyloxy carbonyl chloride (3.0 mL) was then added dropwise and the mixture stirred vigorously overnight. The phases were separated and the aqueous phase extracted with CH₂Cl₂ (3 × 20 mL). The combined organic phases were washed with brine (1× 50 mL), dried (Na₂SO₄), filtered, adsorbed onto Celite and chromatographed (ISCO, 120 g silica column, gradient elution 5-55% EtOAc-hexanes) to afford 4.15 g (80%) of racemic Cbz-pyranylglycine methyl ester. The enantiomers were separated on a Chiracel OD column eluting with 10% EtOH-hexanes. The desired S-enantiomer c elutes first under these conditions.

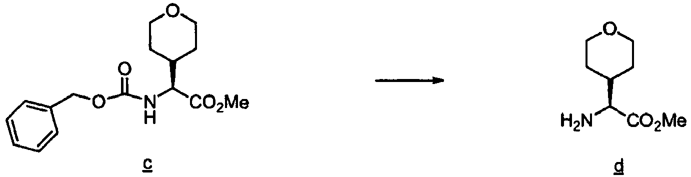

A mixture of (S)-N-Cbz-pyranyl glycine c methyl ester (2.4 g, 7.82 mmol) 10% Pd-C (700 mg), MeOH (80 mL) was maintained under 1 atmosphere of H₂ for 24 h. The mixture was filtered through Celite with MeOH, and concentrated under reduced pressure to afford 1.35 g (100%) of amine d as a colorless oil. Alternatively, pyranyl glycine can be synthesized in enantiopure form following the procedure of Ghosh (Ghosh, A. K.; Thompson, W. J.; Holloway, M. K.; McKee, S. P.; Duong, T. T.; Lee, H. Y.; Munson, P. M.; Smith, A. M.; Wai, J. M.; Darke, P. L.; Zugay, J. A.; Imini, E. A.; Schleif, W. A.; Huff, J. R.; Anderson, P. S. J. Med Chem., 1993, 36, 2300).

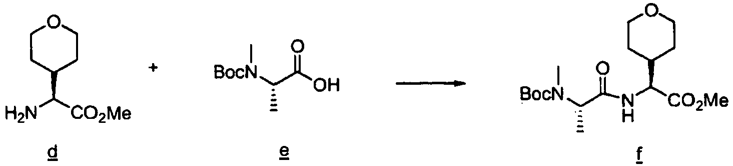

A mixture of amine d (1.35 g, 7.8 mmol), N-Boc-N-methyl alanine e (1.74 g, 8.6 mmol), EDC (1.65 g 8.8 mmol) and MeCN (50 mL) was maintained at rt overnight. The MeCN was removed under reduced pressure, and the residue diluted with EtOAc, washed with 0.5 N HCl (3 × 10 mL), 0.5 N NaOH (3 × 10 mL), dried (MgSO₄) filtered, and concentrated to provide 2.1g (75%) of protected dipeptide f, as a clear oil.

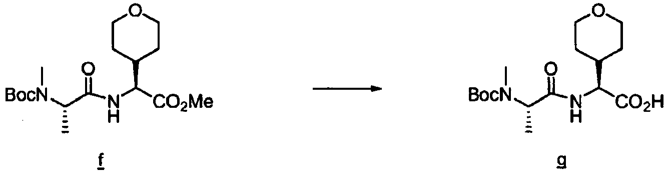

To a 0 °C solution of ester f (2.10 g, 5.86 mmol) and THF (50 mL) were added LiOH·H₂O (1.23 g, 29.3 mmol) and water (2 mL). The mixture was maintained at 0 °C for 2 h, then the cooling bath was removed and the mixture was stirred overnight. Most of the THF was then removed under reduced pressure and the residue was diluted with CH₂Cl₂, washed with 0.5 N HCl, dried (MgSO₄) filtered, and concentrated to provide 1.53 g (78%) of dipeptide N-Boc-N-methyl-L-alanine-L-dehydropyranylglycine g, as a colorless solid.

### Example 9 7-phenyl-2-(pyrrolidin-2-yl)thiazolo[5,4-b]pyridine

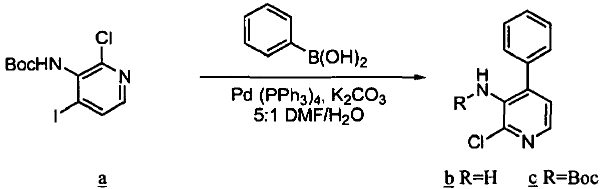

(2-chloro-4-iodo-pyridin-3-yl)-carbamic acid tert-butyl ester a (4.20g, 11.8 mmol), phenyl boronic acid (1.90g, 15.6 mmol), potassium carbonate (2.42g, 17.5 mmol) and tetrakistriphenylphosphine palladium(0) (0.68g, 0.59 mmol) were weighed into a 20 ml microwave vial. The vial was evacuated, then purged with nitrogen gas 3 times. 16.7 ml dry DMF was added, then 3.3 ml of water, which had been degassed by bubbling nitrogen through it overnight. The vial was then capped and microwaved at 130°C for 40 minutes. The resulting solution was poured into 250 ml water and extracted with EtOAc (3x50 ml). The combined organics were dried with MgSO₄ filtered and concentrated. The resulting oil was adsorbed onto silica gel and purified by flash chromatorgraphy (150g SiO₂, 0% to 40% EtOAc in hexanes) to give 2-chloro-3-amino-4-phenyl pyridine b (0.84g, 4.1 mmol, 35%) and the Boc-protected 2- chloro-3-amino-4-phenyl pyridine c (1.74g, 5.7 mmol, 48%) as yellow and white solids, respectively.

### Example 10 7-phenyl-2-((S)-pyrrolidin-2-yl)thiazolo[5,4-c]pyridine

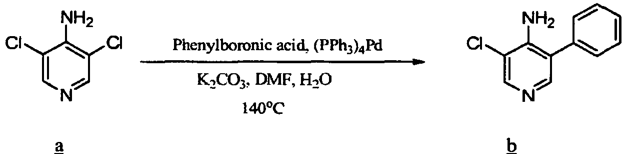

4-amino-3,5-dichloropyridine a (2.0 g, 12.3 mmol), tetrakis(triphenylphosphine)palladium (696 mg, 0.6 mmol), phenylboronic acid (1.9 g, 15.9 mmol) and potassium carbonate (2.2 g, 15.9 mmol) were mixed in a 10 mL microwave vial under N₂ atmosphere. DMF (6 mL) and deoxygenated H₂O (1.2 mL) were added. N₂ was bubbled through the mixture for 5min and the mixture was heated for 20min at 140°C in the microwave. The mixture was diluted with water (30 mL) and extracted with EtOAc (3x20 mL). The combined organic phases were washed with water (50 mL) and brine (50 mL), dried with MgSO₄ filtered and concentrated. The resulting brown oil was adsorbed on silica gel and purified by flash chromatography (SiO₂, 0% to 70% ethyl acetate/hexanes) to afford 970 mg (37%) of b as a colorless oil. MS: m/z = 205 (M+H).

### Example 11 7-phenyl-2-((S)-pyrrolidin-2-yl)thiazolo[5,4-d]pyrimidine

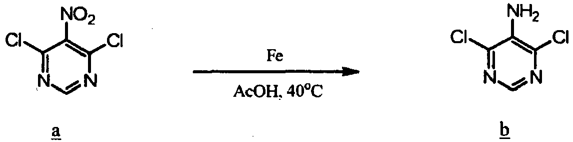

Iron powder (12.5 g, 112 mmol) was added to a suspension of 4,6-dichloro-5-nitropyriinidine a (7.0 g, 36.1 mmol) in acetic acid (70 mL). The mixture was stirred at 40°C for 45min. The mixture was poured onto ice and neutralized by addition of solid sodium bicarbonate. The aqueous phase was extracted with EtOAc (3x200 mL). The combined organic phases were dried with MgSO₄ filtered and concentrated to afford a pale yellow solid. Recrystallization in hot ethyl acetate afforded 3.6 g (61%) of compound b as off-white needles. MS: m/z = 165 (M+H).

### Example 12 2,3-diaminobiphenyl

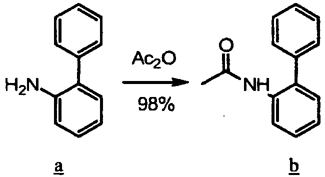

2-Aminobiphenyl a (21.9289g, 130mmol) was dissolved in Ac₂O (30mL, 318mmol) and stirred 10 minutes. An additional portion of Ac₂O (10mL, 106mmol) was added then stirred for 10 more minutes. The sample was poured onto ice. The resulting solid was vacuum filtered and washed with H₂O to give N-acetyl-2-aminobiphenyl b (26.955g, 128mmol, 98%).

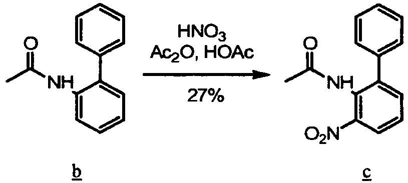

Following the general procedure of Stepan (Stepan, A. H., et al, J. Am. Chem. Soc., 1949, 71, 2438), N-acetyl-2-aminobiphenyl b (7.198g, 34.1mmol), HOAc (6mL), and Ac₂O (5mL) were mixed and heated at 120°C for a few minutes until N-acetyl-2-aminobiphenyl b was dissolved. The sample was cooled to room temperature. HOAc (1.5mL)) was added slowly to 2.3mL of fuming HNO₃ (2.3mL, 54.5mmol) in an ice bath. While maintaining a temperature of less than 26.5°C, 1.5mL) of the HNO₃ mixture was added quickly then the remaining HNO₃ mixture was added drop wise to N-acetyl-2-aminobiphenyl b. The sample was stirred at room temperature for 4 hours then stored at 4°C overnight. The reaction mixture was poured into ice and extracted once with benzene. The benzene layer was stored at 4°C for 1 hour. The resulting solid was vacuum filtered and washed with cold benzene to give N-acetyl-2-amino-3-nitrobiphenyl c (2.346g, 9. 15 mmol, 27%).

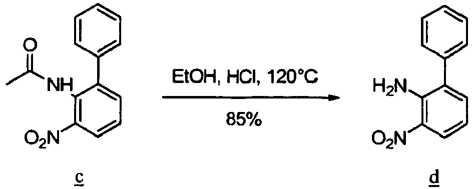

N-Acetyl-2-amino-3-nitrobiphenyl c (1.008g, 3.93mmol), EtOH (19mL, 325mmol), and concentrated HCl (5mL, 5Ommol) were mixed and refluxed at 120°C overnight. The sample was adsorbed onto silica gel and purified by flash chromatography (12g SiO₂, 0-33% EtOAc in hexanes) to give 2-amino-3-nitrobiphenyl d (0.720g, 3.36mmol, 85%)

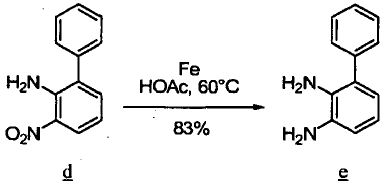

2-Amino-3-nitrobiphenyl d (0.613g, 2.86mmol) was purged under nitrogen for 30 minutes then HOAc (5mL) was added followed by iron powder (0.4895g, 8.76mmol). The sample was heated at 60°C for 30 minutes then HOAc (5mL) was added. The sample was stirred at 60°C for 1 hour then poured into ice. The sample was extracted with EtOAc (3 X 100 mL). The EtOAc extracts were washed with saturated NaHCO₃ (3 X 100 mL. The EtOAc layer was dried over MgSO₄, filtered, and concentrated to give 2,3-diaminobiphenyl e (0.439g, 2.38mmol, 83%).

### Example 13 3-Amino-4-chloro-2-phenylpyridine

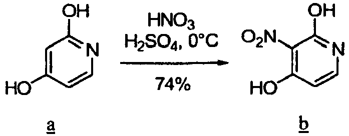

Following the general procedure of Norman (Norman, M. H., et al, J Med. Chem., 2000, 43, 4288), 2,4-dihydroxypyridine (4.931 g, 44.4mmol) and H₂SO₄ (20mL) were combined and cooled to 0°C. HNO₃ (20mL, 444mmol) was added dropwise. The sample was stirred for 30 minutes then poured onto ice. The resulting solid was stored at 4°C for 1 hour then vacuum filtered to give 2.4-dihydroxy-3-nitropyridine (5.143g, 32.9mmol, 74%).

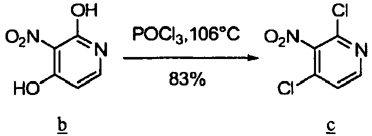

Following the general procedure of Norman (Norman, M. H., et al, J. Med. Chem., 2000, 43, 4288), 2.4-dihydroxy-3-nitropyridine b (2.0013g, 12.9mmol) and POCl₃ (25mL, 268mmol) were combined under nitrogen. The mixture was heated to 106°C and stirred overnight. The sample was concentrated and poured onto ice. The reaction mixture was extracted with EtOAc (3 X 100 mL). The EtOAc extracts were washed with saturated NaCl (1X 100 mL). The EtOAc layer was dried over MgSO₄ and filtered. The crude material was adsorbed onto silica gel, filtered through a plug of silica gel (50%EtOAc in hexanes), and concentrated to give 2,4-dichloro-3-nitropyridine c (2.058g, 10.7mmol, 83%).

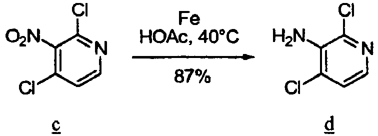

2.4-Dichloro-3-nitropyridine c (2.058g, 10.7mmol) was dissolved in HOAc (10mL) under nitrogen. Iron powder (1.9191g, 34.4mmol) was added. The sample was heated at 40°C for two hours. The reaction mixture was poured onto ice and then NaHCO₃ was added to give a neutral solution. The sample was extracted with EtOAc (3 X 100 mL). The EtOAc extracts were washed with saturated NaHCO₃ (1X 100 mL). The combined aqueous layers were back extracted once with 100 mL EtOAc. The combined EtOAc extracts were dried over MgSO₄, filtered, and concentrated to give 3-amino-2-4-dichloropyridine d (1.510g, 9.26mmol, 87%).

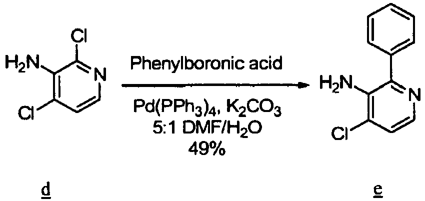

3-Amino-2-4-dichloropyridine d (0.7047g, 4.32mmol), phenylboronic acid (0.5177g, 4.24mmol), K₂CO₃ (0.8023g, 5.80mmol), and Pd(PPh₃)₄ (0.0702g, 0.0607mmol) were combined. The sample was evacuated and purged with nitrogen three times. Dry DMF (2mL) and deoxygenated H₂O (0.4mL) were added. The sample was microwaved at 130°C for 40 minutes. The reaction mixture was diluted with H₂O (50 mL) and extracted with EtOAc (3 X 50 mL). The EtOAc extracts was dried over MgSO₄ and filtered. The crude material was adsorbed onto silica gel and purified by flash chromatography (40g SiO₂, 0-30%EtOAc in hexanes) to give 3-amino-4-chloro-2-phenylpyridine e (0.435g, 2.12mmol, 49%).

### Example 14 N-Boc-protected cyclic sulfonyl amino acid

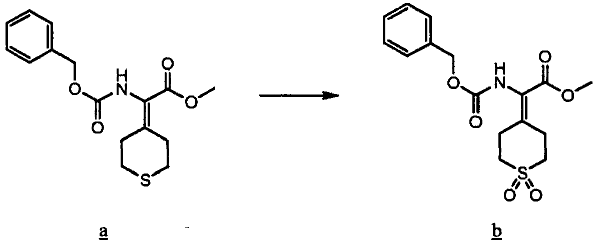

Sulfide a (810 mg, 2.5 mmol), synthesized according to the general procedure of Shieh [Shieh, W-C.; Xue, S.; Reel, N.; Wu, R.; Fitt, J.; Repic, O. Tetrahedron: Asymmetry, 2001, 12, 2421-2425], was dissolved in methanol (25 mL). Oxone (4.5g) was dissolved in deionized water (25 mL). The methanol solution of substrate was cooled to -10 °C, and the aqueous solution of oxone was added to the reaction slowly. The reaction was kept on ice and gradually allowed to warm to room temperature while stirring overnight. Deionized water was used to dilute the reaction to approximately 150 mL, then poured into 90% ethyl acetate-hexanes for extraction. The organic phase was dried (Na₂SO₄), adsorbed onto Celite and purified by chromatography ISCO CombiFlash 40 g column, 5-90% ethyl acetate-hexanes over 30 min to afford 804 mg (2.27 mmol, 91%) of the product sulfone b.

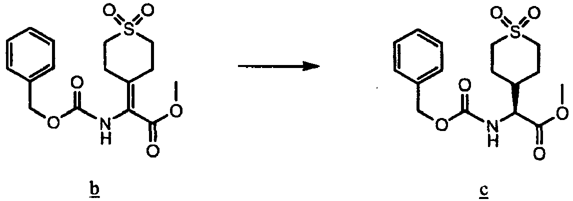

Following the general procedure of Burk [Burk, M. J.; Gross, M. F.; Martinez, J. P. J. Am. Chem. Soc. 1995, 117, 9375-9376.], alkene b (774 mg 2.19 mmol), dry methanol (40 mL), and [(S,S)-Me-BPE-Rh(COD)]⁺OTf⁻ (500 mg, 0.8 mmol) were mixed in a Parr shaker flask purged with nitrogen. The Parr flask was evacuated and subsequently charged to 60 psi with hydrogen gas and shaken vigorously overnight. Methanol was removed under reduced pressure, and crude product was filtered through a small plug of silica gel using ethyl acetate. Evaporation of the solvent yielded 730 mg (2.0 mmol, 94%) of product c with >98% yield.

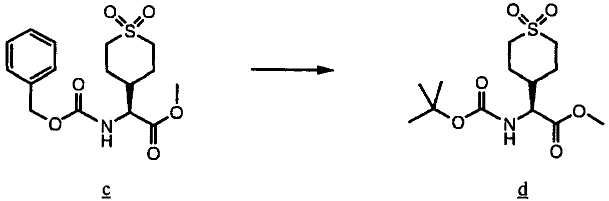

Z-protected amino ester c (804 mg, 2.27 mmol) was dissolved in methanol (16 mL). To this solution was added BOC-anhydride (1.5 g, 6.8 mmol), followed by 20% Pd(OH)₂·C (250 mg). All air was removed from the reaction flask by house vacuum, and the mixture was stirred vigorously for 5 min. The flask was then filled with hydrogen gas and allowed to stir vigorously at room temperature for 6 h. After evacuating the hydrogen atmosphere, the mixture was filtered through Celite using methanol, and crude product d was obtained by evaporation of the solvent (508 mg, 1.56 mmol, 70% yield).

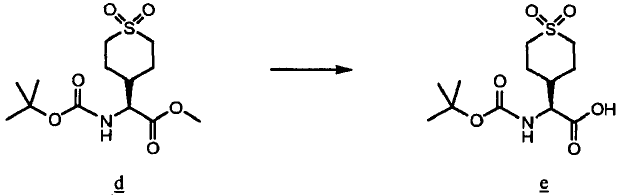

Ester d (508 mg, 1.56 mmol) was dissolved in 8 mL of THF. Deionized water (4 mL) was added, followed by LiOH · H₂O (120 mg, 2.8 mmol). The mixture was stirred at room temperature overnight, acidified using aqueous 1 N HCl and extracted into ethyl acetate (3 X 25 mL). The organic extracts were dried further with Na₂SO₄, filtered and concentrated to give 372 mg (1.21 mmol, 78% yield) of the N-Boc-protected cyclic sulfonyl amino acid e, which was carried on without purification.

### Example 15 N-ethyl-Boc glycine

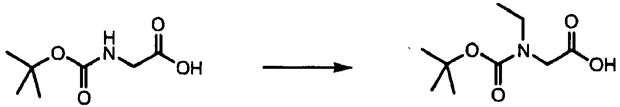

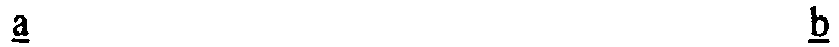

Following the general procedure of Grigg [Blaney, P.; Grigg, R.; Rankovic, Z.; Thornton-Pett, M.; Xu, J. Tetrahedron, 2002, 58, 1719-1737] a roundbottom flask was charged with sodium hydride (480mg 60% dispersion in oil, 12.0 mmol, 4.0 equiv) and purged with nitrogen for 15 min. THF (6.0mL) was added to the flask, and the suspension was cooled to 0 °C using an ice water bath. A separate flask was charged with BOG-glycine a (525 mg, 3.0 mmol), dry THF (6.0 mL) and ethyl iodide (1.0 mL, 12 mmol, 4 equiv). This mixture was added dropwise to the NaH suspension in THF, with vigorous stirring at 0 °C. After 1 h of stirring, the reaction was warmed to room temperature and allowed to stir overnight. The reaction was again cooled to 0 °C, and methanol (4 mL) was added very slowly to quench the excess hydride. Deionized water was added to dilute the mixture, and methanol was removed under reduced pressure. Impurities were extracted into 90% ethyl acetate-hexanes, the aqueous layer was then acidified by adding solid citric acid until the pH reached 2-3. The product was extracted into 90% ethyl acetate-hexanes. This organic layer was dried (Na₂SO₄) and filtered. Removal of the solvents under reduced pressure afforded a quantitative yield of the product N-ethyl-Boc-glycine b.

### Example 16 Boc-fluoro-glycine

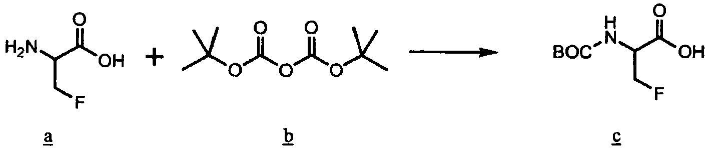

A mixture of 2-amino-3-fluoropropanoic acid a (775 mg, 7.24 mmol) and sodium carbonate (1.69 g, 16.0 mmol) was dissolved in a 1:1 solution of deionized water and THF (15 mL each). To this mixture was added BOC-anhydride b (1.73 g, 7.96 mmol). The mixture was stirred at room temperature overnight, and THF was removed under reduced pressure. The mixture was then acidified to pH 2-3 with saturated aqueous citric acid, and product was extracted into 10% ethyl acetate-dichloromethane. The organic layer was dried (Na₂SO₄), filtered and concentrated under reduced pressure to afford clean Boc-protected amino acid c (1.40 g, 6.7 mmol, 93%) to be used without further purification.

### Example 17 Boc-N-Me-Gly-(cyclohexyl)Gly-OH

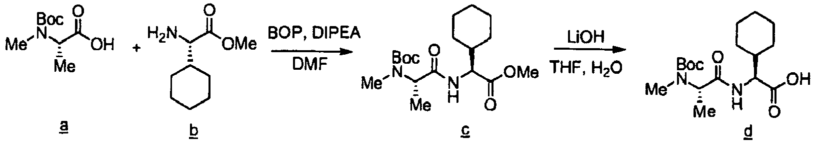

N-Me,Boc-Ala a (4.7 g, 23.1 mmol), Chg-OMe b (4 g, 19.2 mmol), BOP (10.2 g, 23.1 mmol) and DIPEA (7.4 ml, 42.3 mmol) were stirred in 15 ml DMF for 4 hr. EtOAc was added to the solution and the organic layer was washed with saturated aqueous NaHCO₃ twice, with brine twice and dried over MgSO₄ and concentrated.

The crude residue containing c was dissolved in 30 ml THF and lithium hydroxide (1.7 g, 40.8 mmol) in 30 ml water was added and stirred for 1.5 hours. The solution was evaporated to remove the THF and the solution was acidified with aqueous citric acid (approx >2 equivalents) to pH ~3. The solution was extracted twice with EtOAc, the EtOAc layers were combined and washed twice with water and brine, dried over MgSO₄ and concentrated. The acid Boc-N-Me-Gly-(cyclohexyl)Gly-OH d then was purified by HPLC to provide a white fluffy solid after lyophilization.

### Example 18 Fmoc-N-Me('Bu)Gly-OH

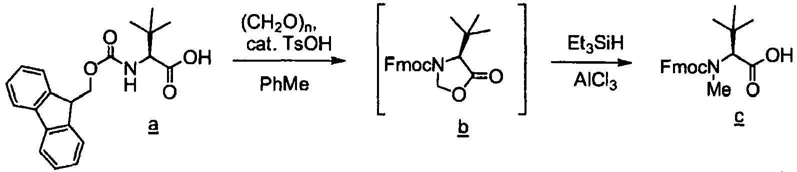

Fmoc-L-α-*t*-butylglycine a (2.0 g, 5.7 mmol) was taken up in anhydrous toluene (110 mL) in a 250-mL flask equipped with a Dean-Stark apparatus and a reflux condenser. Paraformaldehyde (1.12 g) was added followed by *p*-toluenesulfonic acid monohydrate (0.67 mmol, 127 mg). The resulting mixture was heated to 112°C and stirred 1 h. After this period, the flask was cooled to room temperature and the reaction mixture was diluted with Et₂O (200 mL). This solution was washed with saturated aqueous NaHCO₃ solution (2 x 20 mL) and brine (20 mL). The organic portion was dried over MgSO₄, filtered and concentrated in vacuo to provide a crude residue containing the oxazolidinone b. This residue was dissolved in CH₂Cl₂ (114 mL) and aluminum chloride (11.2 mmol, 1.49 g) was added. The reaction mixture immediately turned a green color. Triethylsilane (11.4 mmol, 1.82 mL) was subsequently added and the resulting yellow mixture was stirred 5 h at ambient temperature. The reaction was quenched by the addition of 1 N HCl aqueous solution (35 mL). The mixture was further diluted with H₂O (100 mL) and the biphasic mixture was partitioned. The aqueous layer was extracted with CH₂Cl₂ (2 x 50 mL); the combined organic layers were washed sequentially with 1 N HCl (30 mL) and saturated aqueous NaHCO₃ solution (30 mL) and brine (30 mL). The organic portion was dried over MSO₄, filtered and concentrated. The residue was purified by ISCO chromatography (0 to 50% EtOAc/Hexanes, slow-gradient) to provide Fmoc-*N*-Me(*^{t}*Bu)Gly-OH c as a white flaky solid (1.46 g, 70% yield over 2 steps). LC/MS analysis confirmed the identity of the desired product (MW = 367.4, found M+H⁺ = 368.1).

### Example 19 (reference example) (S)-tert-butyl 1-(4-(4-fluoronaphthalen-1-yl)thiazol-2-yl)-2-(4-methoxybenzylthio)-2-methylpropylcarbamate

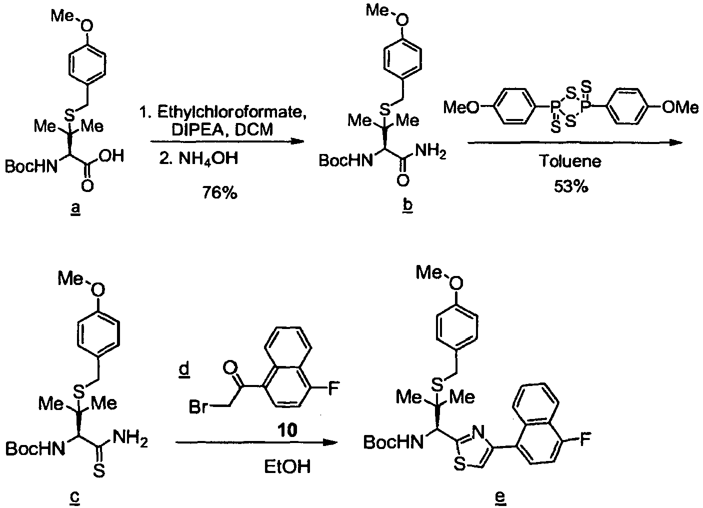

Penicillamine derivative a (2.0 g, 5.4 mmol) was dissolved in 30 ml DCM and 2.4 ml DIPEA was added. The solution was cooled to 0 °C and 2.3 ml chloroethylformate was added dropwise. The reaction was warmed to room temperature over one hour and then cooled to 0 °C. To this solution 30 ml of 30% NH₄OH was added and the reaction was stirred for two hours. The layers were separated and the DCM layer was extracted once each with 50 ml 0.5 N NaOH, water, and brine and then dried with Na₂SO₄. The amide b (1.5 g, 76%) was isolated using SiO₂ chromotagraphy with an ethyl acetate/hexanes solvent system. Product identity was confirmed by electrospray mass spectrometry (M+H⁺ = 369.1).

Amide b (1.5 g, 4.1 mmol) was dissolved in 15 ml toluene and 1.0 g (2.5 mmol) Lawesson's reagent was added. The reaction was heated to 65 °C under N₂ atmosphere for 4 h. The reaction was dry loaded onto celite and thioamide c (850 mg, 53%) was isolated using SiO₂ chromotagraphy with an ethyl acetate/hexanes solvent system. Product mass indicated M+H⁺ = 385.1 by electrospray mass spectrometry.

Thioamide c (850 mg, 2.2 mmol) was combined with the bromide d (710 mg, 2.7 mmol) in refluxing EtOH. Thiazole e was isolated by reverse phase HPLC. Product mass indicated M+H⁺ = 453.1 by electrospray mass spectrometry.

### Example 20 (R)-tert-butyl 3-(acetamidomethylthio)-1-(2,2-diphenylethylamino)-3-methyl-1-oxobutan-2-ylcarbamate

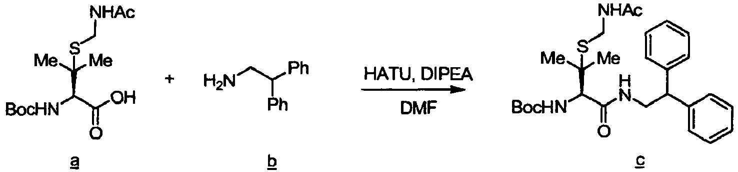

To a stirred solution of a (360 mg, 1.13 mmol) in 5 mL dry DMF was added HATU (428 mg, 1.13 mmol), diphenylethylamine b (171 mg, 0.87 mmol) and DIPEA (365 µL, 2.1 mmol). The reaction was stirred at room temperature under N₂ for 2 hours and then diluted with EtOAc, washed 2x with saturated NaHCO₃, washed 2x with brine, dried with MgSO₄ and concentrated. This yielded the compound c after ISCO chromatography. MS = 500.4 (M+H⁺).

The following P3-P4 intermediate was prepared using the above procedure:

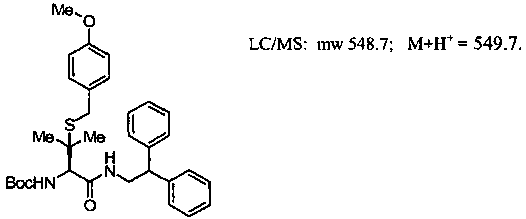

### Example 21 (reference example) (R)-tert-butyl-3-(4-methoxybenzylthio)-3-methyl-1-(3-methyl-1-phenyl-1H-pyrazol-5-ylamino)-1-oxobutan-2-ylcarbamate

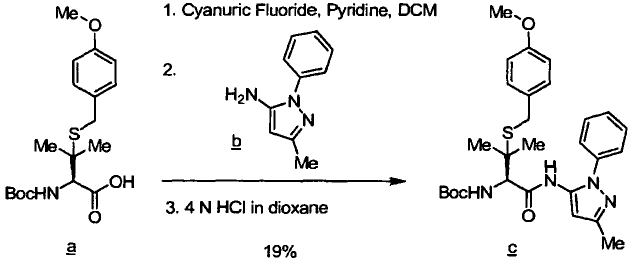

Boc-Pen(PMB)-OH a (540 mg, 1.46 mmol) was dissolved in dry DCM (5 mL) and cooled to 0°C, pyridine (118 µL, 1.46 mmol) and cyanuric fluoride (123 µL, 1.46 mmol) were added. The mixture was allowed to warm to room temperature, stirred for 45 minutes and then diluted with DCM, washed with brine and dried with Na₂SO₄, filtered and concentrated. The intermediate was dissolved in dry DCM (5 mL), cooled to 0°C followed by the addition of 5-amino-3-methyl-1-phenyl pyrazole b (169 mg, 0.97 mmol), allowed to warm to room temperature and stirred for 12 hours and then diluted with DCM, washed with brine, dried with Na₂SO₄ and concentrated. The residue was dissolved in 4N HCl/dioxanes (10 mL) and stirred at room temperature for 30 minutes. The solvent was removed and purification by preparative HPLC gave compound c. MS = 425.5 (M+1).

### Example 22 (reference example) EDC/HOBt coupling of P3 and P4 units

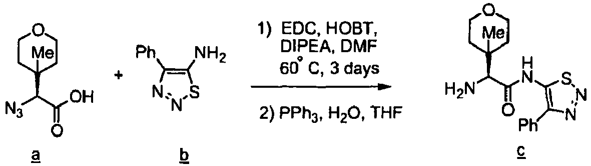

Azido compound a (360 mg, 1.8 mmol) was dissolved in DMF (3.5 mL) and 4-phenyl-1,2,3-thiadiazole-5-amine b (3.6 mmol, 620 mg) was added. Diisopropylethylamine (1.8 mmol, 310 µL), 3-hydroxybenzotriazole (1.8 mmol, 241 mg) and 1-(3-dimethylaminopropyl)-3-ethyl-carbodiimide (1.8 mmol, 341 mg) were then added to the mixture and the resulting reaction mixture was heated to 60 °C under a nitrogen atmosphere for 72 h. The reaction was cooled to r.t. and quenched with saturated aqueous ammonium chloride solution (10 mL). Further dissolution with EtOAc (100 mL) and water (50 mL) was carried out. The aqueous layer was extracted with EtOAc (50 mL) and the combined organic layers were washed with saturated sodium bicarbonate solution (2 x 30 mL) and brine (30 mL) and then dried over MgSO₄, filtered and concentrated. The crude residue was purified by ISCO chromatography (0 to 50% EtOAc/Hexanes) to give the product as an off-white solid (630 mg, 98% yield). This material (630 mg, 1.75 mmol) was dissolved in THF (20 mL) and PPh₃ (3.6 mmol, 940 mg) was added. After stirring for 3 h at r.t., H₂O (18.0 mmol, 330 µL) was added all at once and the resulting mixture was stirred 16 h. The mixture was quenched with saturated aqueous sodium bicarbonate solution (10 mL) and then diluted with H₂O (100 mL) and EtOAc (100 mL). The layers were partitioned and the aqueous layer was extracted with EtOAc (50 mL). The combined organics were dried with MgSO₄, filtered and concentrated. LC/MS analysis indicates presence of the desired compound (S)-2-amino-2-(4-methyltetrahydro-2H-pyran-4-yl)-N-(4-phenyl-1,2,3-thiadiazol-5-yl)acetamide c (MW = 332.4, found M+H⁺ = 333.5) along with residual triphenylphosphine oxide.

The following P3 and P4 unit was also coupled using the above EDC/HOBt procedure above:

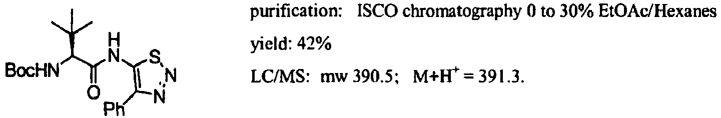

### Example 23 (reference example) (S)-tert-butyl-3,3-dimethyl-1-oxo-1-(4-phenyl-2-(pyrazin-2-yl)thiazol-5-ylamino)butan-2-ylcarbamate

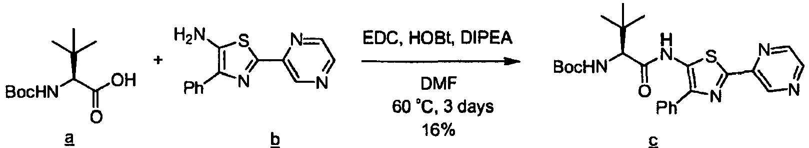

*N*-Boc-L-α-tert-butylglycine a (0.38 g, 0.0016 mol) was dissolved in *N,N*-dimethylformamide (0.98 mL, 0.013 mol) and 4-phenyl-2-(pyrazin-2-yl)thiazol-5-amine b (410 mg, 0.0016 mol) was added followed by *N*-(3-dimethylaminopropyl)-*N'*-ethylcarbodiimide hydrochloride (310 mg, 0.0016 mol) and 1-hydroxybenzotriazole (220 mg, 0.0016 mol) and finally *N,N*-diisopropylethylamine (380 uL, 0.0022 mol). The reaction was heated to 60 °C, stirred for 3 d and then quenched by the addition of a saturated aqueous solution of sodium bicarbonate. The aqueous layer was extracted with EtOAc, and the combined organic layers were dried over MgSO₄, filtered was evaporated. The crude residue was purified by ISCO chromatography (40 g column, 0 to 50% EtOAc/Hexanes) to give 119 mg of the product c (16% yield).

### Example 24 (reference example) (S)-3,3-dimethyl-2-(methylamino)-N-(4-phenyl-1,2,3-thiadiazol-5-yl)butanamide

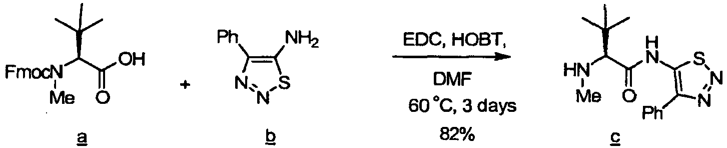

Fmoc-*N*-Me-*t*-butylglycine a (709 mg, 1.93 mmol) was dissolved in DMF (1.72 mL) and 4-phenyl-1,2,3-thiadiazole-5-amine b (1.83 mmol, 318 mg) was added. 1-hydroxybenzotriazole (1.95 mmol, 264 mg) and 1-(3-dimethylaminopropyl)-3-ethyl-carbodiimide (1.95 mmol, 377 mg) were then added to the mixture. The resulting reaction mixture was heated to 60 °C under a nitrogen atmosphere for 3 days. The reaction was cooled to r.t. and quenched with saturated aqueous ammonium chloride solution (10 mL). Further dissolution with EtOAc (100 mL) and water (50 mL) was carried out. The aqueous layer was extracted with EtOAc (50 mL) and the combined organic layers were washed with saturated sodium bicarbonate solution (2 x 30 mL) and brine (30 mL) and then dried over MgSO₄, filtered and concentrated. The crude residue was purified by ISCO chromatography (0 to 100% EtOAc/Hexanes) to give 483 mg of product c (82% yield).

### Example 25 (reference example) PyAOP/collidine coupling of P3 and P4 units

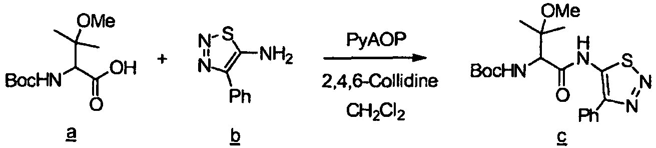

PyAOP (1.62 mmol, 845 mg) was added in one portion to a dichloromethane (7 mL) solution containing the acid a (1.6 mmol, 400 mg), 4-phenyl-1,2,3-thiadiazole-5-amine b (2.43 mmol, 430 mg) and 2,4,6-collidine (3.24 mmol, 428 µL) at 0 °C. The reaction mixture was allowed to gradually warm to r.t. over 20 h. and then was poured into a separatory funnel containing EtOAc (30 mL), washed sequentially with 10% aq. citric acid (10 mL), saturated aq. NaHCO₃ (10 mL) and brine (10 mL) and then dried over MgSO₄, filtered and concentrated in vacuo. The resulting crude was purified by ISCO chromatography (0 to 50% EtOAc/Hexanes) to give 520 mg of product c (79% yield) as a yellow oil. LC/MS analysis confirmed the identity of the desired product (MW = 406.5, found M+H⁺ = 407.7).

The following P3-P4 intermediates were prepared using the above PyAOP/collidine procedure above (reference examples):

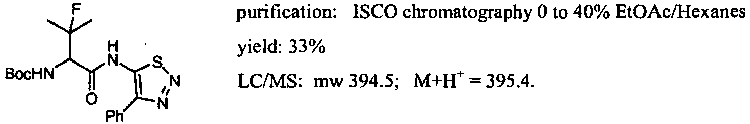

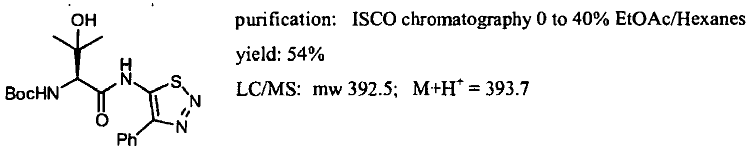

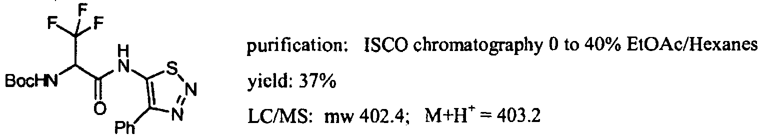

### Example 26 (reference example) FMOC-protected dimethylpenicillamine P3-P4 intermediate

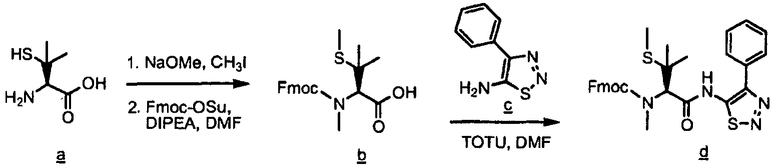

To a stirred solution of (R)-2-amino-3-mercapto-3-methylbutanoic acid a (500 mg, 3.35 mmol) in 13.1 mL 0.5 N NaOMe in MeOH was added CH₃I (784 mg, 5.56 mmol) and the reaction ws stirred at room temperature overnight. The solvent was removed and then redissolved in 5 mL dry DMF followed by addition of DIPEA (600 µL, 3.44 mmol) and Fmoc-OSu (1.1 g, 3.30 mmol) and the reaction was stirred under N₂ at room temperature overnight. DMF was removed under reduced pressure, DCM was added and then the reaction was washed 1x with H₂O, 2X with 10% citric acid and brine, dried with MgSO₄ and then concentrated. The material was purified via flash chromatography, 0-10% MeOH in H₂O to give Fmoc-N-methyl intermediate b. MS = 401.2 (M+1).

4-phenyl-1,2,3-thiadiazol-5-amine c (145 mg, 0.82 mmol) and TOTU (269 mg, 0.82 mmol) were combined in 3 mL dry DMF and intermediate b (327 mg, 0.82 mmol) and DIPEA were added and the reaction was stirred under N₂ at room temperature overnight. The reaction was then diluted with EtOAc, washed 2x with saturated NaHCO₃, 2x with H₂O and 1x with brine, dried with MgSO₄ and then concentrated. Purified via preparative HPLC to gave intermiate d. MS = 559.2 (M+1).

### Example 27 (R)-tert-butyl 1-(2,2-diphenylethylamino)-3-methyl-3-(methylthio)-1-oxobutan-2-ylcarbamate

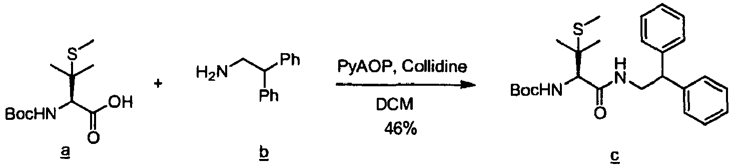

To a solution of a (105 mg, 0.24 mmol), 2,2-diphenylethylamine b (44 mg, 0.22 mmol), and 2,4,6-collidine (64 µL, 0.48 mmol) in dichloromethane (3 mL) at 0° C was added PyOAP (125 mg, 0.24 mmol) in one portion. The reaction was allowed to warm to r.t. overnight. The reaction mixture was poured into a separatory funnel containing EtOAc (15 mL) and washed with 10% citric acid (15 mL), saturated NaHCO₃ (15 mL), and brine (15 mL). The combined aqueous layers was extracted with EtOAc (3 × 10 mL) and the combined organic layers was dried over MgSO₄, filtered, and concentrated in vacuo. ISCO chromatography (0 to 100% hexanes/EtOAc, slow gradient) gave 50 mg of c (46% yield) as a white solid. LCMS analysis confirmed the identity of the desired product (MW = 422.4, found M+H⁺ = 443.6).

The following P3-P4 intermediates were prepared according to the above procedure:

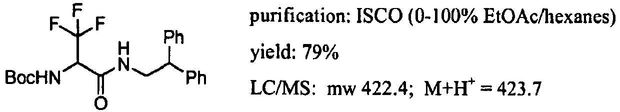

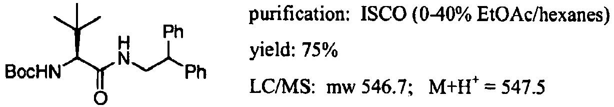

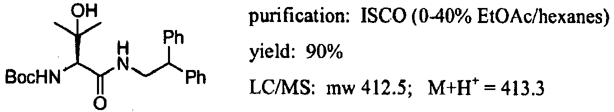

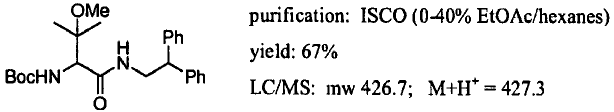

### Example 28 (reference examples) compounds 1 to 3 and 5 to 6

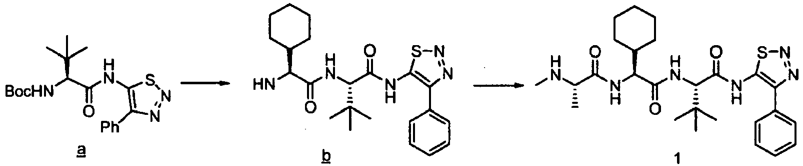

Thiadiazole compound a (75 mg, 0.19 mmol) was treated with 10 ml 4 N HCl/dioxane for 30 minutes and the solvent was removed. Boc-L-cyclohexylglycine (53 mg, 0.20 mmol), HATU (78 mg, 0.20 mmol), DIPEA (72 ul, 0.40 mmol) were combined in 2 ml DMF and stirred overnight at room temp. Standard workup: Ethyl acetate was added and organic layer washed twice with aqueous sodium bicarbonate, washed twice with brine, dried over MgSO₄ and concentrated. The residue of intermediate b was a single peak with the correct mass by LC/MS and was used in the next step without purification. This intermediate b was then treated with 10 ml 4 N HCl/dioxane for 30 minutes and the solvent removed. Boc-N-methylalanine (42 mg, 0.20 mmol), HATU (78 mg, 0.20 mmol) and DIPEA (72 ul, 0.40 mmol) were combined in 2 ml DMF and stirred for 3 hours at room temp. Standard workup: Ethyl acetate was added and organic layer washed twice with aqueous sodium bicarbonate, washed twice with brine, dried over MgSO₄ and concentrated. The resulting mixture was treated with 10 ml 4 N HCl/dioxane for 30 minutes and the solvent was removed. The residue was purified by HPLC to yield 9 mg (8% yield over 5 steps) of compound 1 after solvent lyophilization. The identity of the structure was assigned based on LC/MS (MW = 514.7, found M+H⁺ = 515.9).

The following compounds were prepared according to the above procedure from the appropriate intermediates. For compounds prepared from intermediates incorporating a racemic P3 residue, the final compound was separated from the diastereomeric mixture by chiral HPLC under the following conditions: 25 to 45% acetonitrile in 30 min at 75 mL/min using a 250 X 30 mm Phenomenex C18 column. The diastereomer having activity according to the biological assays herein were assigned the stereochemistry of the final compound based on the stereochemical orientation known to be required for activity.

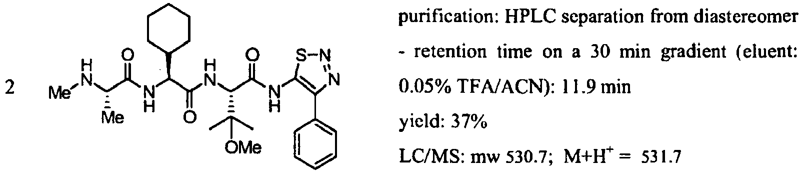

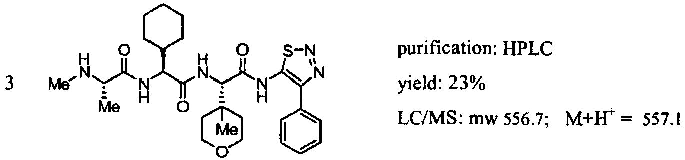

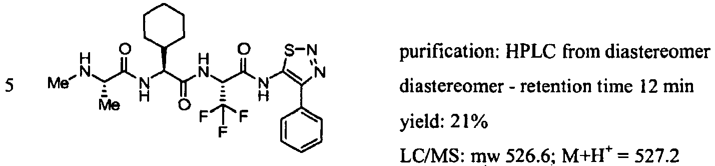

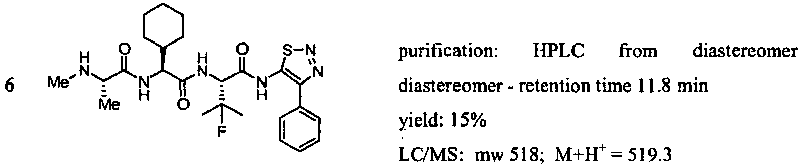

### Example 29 compound 8

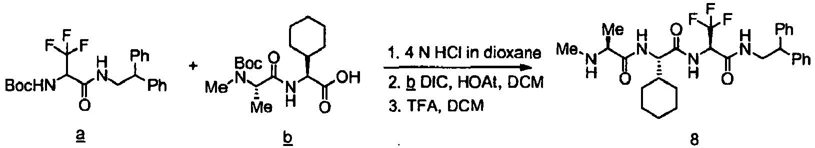

Compound a (1.08 g, 2.55 mmol) was treated with 4N HCl in 1,4-dioxane (96 mL) and stirred at rt for I h. The reaction was quenched by a dropwise addition of satd NaHCO₃ and basified further with 1N NaOH until pH 8-9 was achieved. The reaction mixture was extracted with EtOAc (4 × 25 mL) and the combined organic layers was dried over MgSO₄, filtered, and concentrated in vacuo to give 1.02 g (quantitative yield) of the crude product as a yellow solid. To a 350 mg (1.09 mmol, 1.0 equiv) portion of the crude residue dissolved in dichloromethane (16 mL) was added Boc(Me)AlaChg dimer b (562 mg, 1.64 mmol) and HOAt (223 mg, 1.64 mmol). The mixture was stirred at rt for 5 min and DIC (256 µL, 1.64 mmol, 1.5 equiv) was added. The reaction mixture was stirred at rt overnight and then quenched with satd NaHCO₃ (20 mL) and the aqueous layer was extracted with dichloromethane (3 × 10 mL), dried over MgSO₄, filtered and concentrated in vacuo. ISCO chromatography gave 438 mg (62%) of the Boc protected compound as a white powdery solid. (LC/MS MW = 646.7, found M+H⁺ = 647.4). To a solution of the Boc-protected compound (438 mg, 0.677 mmol, 1.0 equiv) in dichloromethane (16 mL) was added TFA (16 mL) and the solution was stirred at rt for 30 min and then concentrated in vacuo to give final compound 2. LCMS analysis confirmed the identity of the desired product (MW = 546.6, found M+H⁺ = 547.3).

### Example 30 (reference example) compound 20 (R)-2-((S)-2-cylohexyl-2-((S)-2-(methylamino)propanamido)-acetamido)-3-methyl-3-(methylthio)-N-(4-phenyl-1,2,3-thiadiazol-5-yl)butanamide

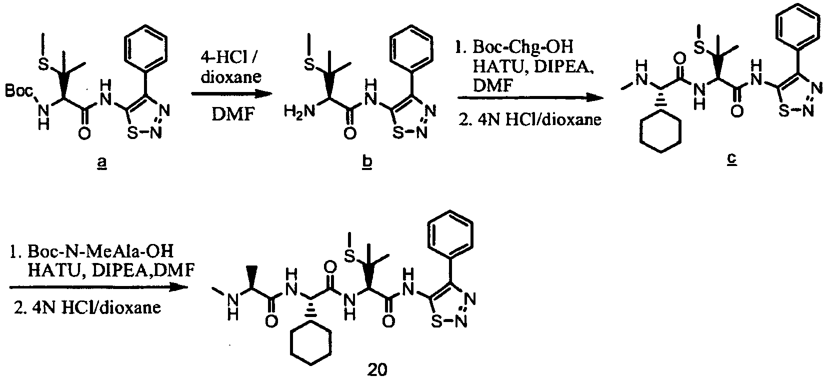

Compound a (40 mg, 0.09 mmol) was dissolved in 10 mL of 4N HCl/dioxane and stirred for 30 minutes to give compound b.

Boc-Chg-OH (30 mg, 0.12 mmol) and HATU (45 mg, 0.12 mmol) were dissolved in 1.5 mL DMF and added to compound b followed by the addition of diisopropylethylamine (42 µL, 0.24 mmol). The reaction was stirred at room temperature under N₂ for 2 hours. Diluted with EtOAc, washed 2x with saturated NaHCO₃, washed 2x with brine, dried with MgSO₄ and concentrated. The concentrate was dissolved in 4N HCl/dioxane (10 mL) and stirred at room temperature for 30 minutes. The solvent was removed to give compound c. MS = 462.2 (M+1) Boc-NMeAla-OH (24 mg, 0.12 mmol) and HATU (45 mg, 0.12 mmol) were dissolved in 1.5 mL DMF and added to compound c followed by the addition of diisopropylethylamine (42 µL, 0.24 mmol). The reaction was stirred at room temperature under N₂ for 2 hours. Diluted with EtOAc, washed 2x with saturated NaHCO₃, washed 2x with brine, dried with MgSO₄ and concentrated. The concentrate was dissolved in 4N HCl/dioxane (10 mL) and stirred at room temperature for 30 minutes. The solvent was removed and the product purified via preparative HPLC to give compound 20. MS = 547.0 (M+1).

### Example 31 (reference example) compound 23 (R)-2-((S)-2-cyclohexyl-N-methyl-2-((S)-2-(methylamino)-propanamido)acetamido)-3-methyl-3-(methylthio)-N-(4-phenyl-1,2,3-thiadiazol-5-yl)butanamide

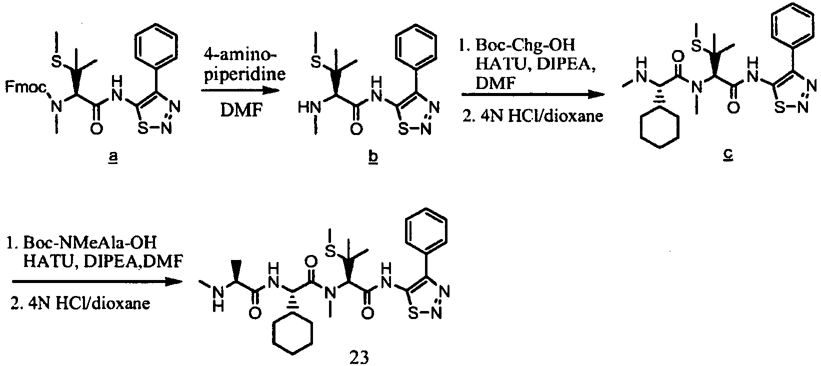

To a stirred solution of a (55 mg, 0.1 mmol) in 5 mL DMF was added 4-aminomethylpiperidine (60 µL, 0.5 mmol). The reaction was complete after 3 hours and purified by Preparative HPLC to give b. MS = 337.0 (M+1).

Boc-Chg-OH (24 mg, 0.09 mmol) and HATU (36 mg, 0.09 mmol) were dissolved in 1.5 mL DMF and added to compound b (26 mg, 0.08 mmol) followed by the addition of diisopropylethylamine (33 µL, 0.19 mmol). The reaction was stirred at room temperature under N₂ for 2 hours. Diluted with EtOAc, washed 2x with saturated NaHCO₃, washed 2x with brine, dried with MgSO₄ and concentrated. The concentrate was dissolved in 4N HCl/dioxane (10 mL) and stirred at room temperature for 30 minutes. The solvent was removed to give compound c. MS = 476.2 (M+1) Boc-NMeAla-OH (19 mg, 0.09 mmol) and HATU (34 mg, 0.09 mmol) were dissolved in 1.5 mL DMF and added to compound c followed by the addition of diisopropylethylamine (33 µL, 0.19 mmol). The reaction was stirred at room temperature under N₂ for 2 hours. Diluted with EtOAc, washed 2x with saturated NaHCO₃, washed 2x with brine, dried with MgSO₄ and concentrated. The concentrate was dissolved in 4N HCl/dioxane (10 mL) and stirred at room temperature for 30 minutes. The solvent was removed and the product purified via preparative HPLC to give the final compound. MS = 561.0 (M+1).

### Example 32 (reference example) compound 24 (S)-2-((S)-2-cyclohexyl-N-methyl-2-((S)-2-(methylamino)propan-amido)acetamido)-3,3-dimethyl-N-(4-phenyl-1,2,3-thiadiazol-5-yl)butanamide

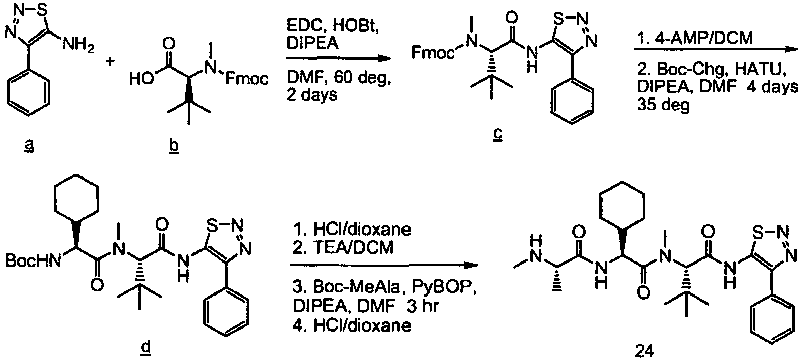

Fmoc-L-methyl t-butylglycine b (416 mg, 1.128 mmol), 4-phenyl-1,2,3,-thiadiazol-5-amine a (100 mg, 0.564 mmol), EDC (204 mg, 1.064 mmol), HOBt (144 mg, 1.064 mmol), DIPEA (492 ul, 2.82 mmol) were combined and stirred for 2 days in 2 ml DMF at 60 °C. Ethyl acetate and saturated aqueous NaHCO₃ were added. The aqueous layer was separated and extracted with ethyl acetate. Organic layers were combined and washed with aqueous NaHCO₃ and brine. Organic layer was dried over MgSO₄ and concentrated to a brown residue. Pure compound c was obtained by flash chromatography. Calculated mass 526.6, found 527.2.

Compound c (76 mg, 0.144 mmol) was treated with 4-aminomethylpiperidine (110 ul, 1.44 mmol) in 10 ml DCM for 1 hour. The solution was evaporated and a standard workup was done and the residue was purified by HPLC. Standard workup: Ethyl acetate was added and organic layer washed twice with aqueous sodium bicarbonate, washed twice with brine, dried over MgSO₄ and concentrated. The purified deprotected residue (32 mg, 0.105 mmol) was reacted with Boc-L-cyclohexylglycine (30 mg, 0.116 mmol), HATU (44 mg, 0.116 mmol) and DIPEA (40 ul, 0.232 mmol) in 2 ml DMF at 35 °C for 4 days. A standard workup was done as described above and compound 2 was purified by HPLC. Calculated mass 543.7, found 544.3.

Compound 2 (5 mg, 0.0092 mmol) was treated with 10 ml 4N HCl/dioxane for 30 min, neutralized with TEA (4 ul, 0.0184 mmol) and reacted with Boc-L-Methylalanine (4 mg, 0.0184 mmol), PyBOP (10 mg, 0.0184 mmol) and DIPEA (4 ul, 0.0184 mmol) in 2 ml DMF for 3 hours. A standard workup was done and the concentrated residue was treated with 10 ml 4N HCl/dioxane for 30 min, concentrated and purified by HPLC to yield 4.0 mg (7%) of the final compound. Calculated mass 528.7, found 529.3.

### Example 33 (reference example) compound 4 (S)-2-((S)-2-cyclohexyl-2-((S)-2-methylamino)propanamido)-acetamido)-3-methyl-N-(4-phenyl-1,2,3-thiadiazol-5-yl)butanamide

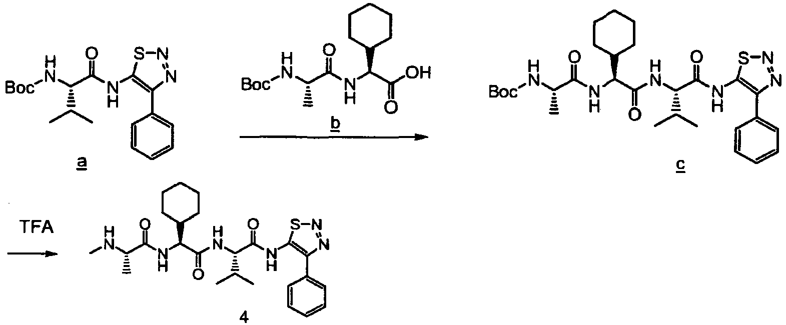

(S)-tert-butyl 3-methyl-1-oxo-1-(4-phenyl-1,2,3-thiadiazol-5-ylamino)butan-2-ylcarbamate a (0.7 g, 2 mmol) was diluted with 4 M of HCl in 1,4-dioxane (46 mL) and stirred at r.t. 30 mins. The reaction mixture was concentrated in vacuo. The residue was then taken up in dichloromethane and (S)-2-((S)-2-(tert-butoxycarbonylamino)propanamido)-2-cyclohexylacetic acid b (0.96 g, 2.8 mmol) was added followed by 1-hydroxy-7-azabenzotriazole (380 mg, 2.8 mmol) and stirred together for 5 min before the addition of N,N'-diisopropylcarbodiimide (440 uL, 2.8 mmol). The resulting reaction mixture was stirred at r.t. overnight and worked up and purified by ISCO chromatography (0-40% EtOAc/Hexanes) to provide the compound c (1.0 g, 30% yield over the two steps).

Compound c (307 mg, 0.51 mmol) was dissolved in DCM (4 mL) and treated with TFA (4 mL, 100 equiv) and stirred at room temperature for 1 hour. Concentration in vacuo and purification by HPLC gave the final compound 4 (S)-2-((S)-2-cyclohexyl-2-((S)-2-(methylamino)propanamido)-acetamido)-3-methyl-N-(4-phenyl-1,2,3-thiadiazol-5-yl)butanamide (314 mg, 56% yield). Example 34 IAP inhibition assays

The following fluorescence polarization experiments used a chimeric BIR domain referred to as MLXBIR3SG in which 11 of 110 residues correspond to those found in XIAP BIR3, while the remainder correspond to ML-IAP BIR. The chimeric protein MLXBIR3SG was shown to bind and inhibit caspase-9 significantly better than either of the native BIR domains, but bound Smac-based peptides and mature Smac with affinities similar to those of native ML-IAP-BIR. The improved caspase-9 inhibition of the chimeric BIR domain MLXBIR3SG has been correlated with increased inhibition of doxorubicin-induced apoptosis when transfected into MCF7 cells.

### MLXBIR3SG sequence:

### TR-FRET Peptide Binding Assay

Time-Resolved Fluorescence Resonance Energy Transfer competition experiments with the compounds of the invention are performed on the Wallac Victor2 Multilabeled Counter Reader (Perkin Elmer Life and Analytical Sciences, Inc.) according to the procedures of Kolb et al (Journal of Biomolecular Screening, 1996, 1(4):203). A reagent cocktail containing 300 nM his-tagged IAP (or BIR construct thereof such as MLXBIR3SG); 200 nM biotinylated SMAC peptide (AVPI); 5 µg/mL anti-his allophycocyanin (XL665) (CISBio International); and 200 ng/mL streptavidin-europium (Perkin Elmer) is prepared in reagent buffer (50 mM Tris [pH 7.2], 120 mM NaCl, 0.1% bovine globulins, 5mM DTT and 0.05% octylglucoside). (Alternatively, this cocktail can be made using europium-labeled anti-His (Perkin Elmer) and streptavidin-allophycocyanin (Perkin Elmer) at concentrations of 6.5 nM and 25 nM, respectively). The reagent cocktail is incubated at room temperature for 30 minutes. After incubation, the cocktail is added to 1:3 serial dilutions of an antagonist compound (starting concentration of 50 µM) in 384-well black FIA plates (Greiner Bio-One, Inc.). After a 90 minute incubation at room temperature, the fluorescence is read with falters for the excitation of europium (340 nm) and for the emission wavelengths of europium (615 nm) and a allophycocyanin (665 nm). Antagonist data are calculated as a ratio of the emission signal of allophycocyanin at 665 nm to that of the emission of europium at 615 nm (these ratios are multiplied by a factor of 10,000 for ease of data manipulation). The resulting values are plotted as a function of antagonist concentration and fit to a 4-parameter equation using Kaleidograph software (Synergy Software, Reading, PA). Indications of antagonist potency are determined from the IC₅₀ values.

### Fluorescence Polarization Peptide Binding Assay

Polarization experiments were performed on an Analyst HT 96-384 reader(Molecular Devices Corp.) in order to determine dissociation constants (K_{d}) between IAP protein BIR domains and the fluorescent probe. Samples for fluorescence polarization affinity experiments were prepared by addition of serial dilutions of IAP BIR domains (C-IAP1 BIR3, C-IAP-2 BIR3, ML/X-IAP chimera MLXBIR3SG and X-IAP BIR3) in polarization buffer (50 mM Tris [pH 7.2], 120 mM NaCl, 1% bovine globulins 5mM DTT and 0.05% octylglucoside) to 5-carboxyflourescein-conjugated AVPdi-Phe-NH₂ (AVP-diPhe-FAM) at 5 nM final concentration.

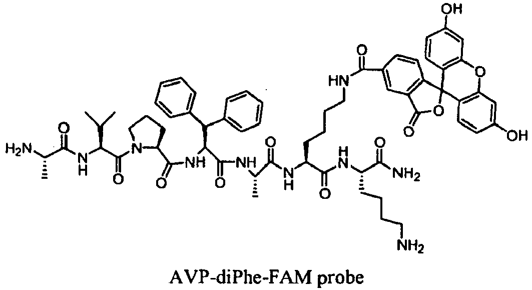

The reactions were read after an incubation time of 1 hour at room temperature with standard cutoff filters for the fluorescein fluorophore (λₑₓ = 485 nm; λₑₘ = 530 nm) in 384-well black plates (Molecular Devices Corp.). Fluorescence polarization values were plotted as a function of the protein concentration, and the effective concentration 50 (EC₅₀) values were obtained by fitting the data to a 4-parameter equation using Kaleidagraph software (Synergy software, Reading, PA). The apparent *K*_{d} values were determined from the EC₅₀ values. Inhibition Constants (*K*ᵢ values) for the antagonists were determined by the addition of 0.06 µM MLXBIR3SG, 0.5 µM X-IAP BIR3, 0.2 µM C-IAPI BIR3 or 0.4 µM C-IAP-2 BIR3 to wells containing 5 nM of the AVP-diPhe-FAM probe as well as 1:3 serial dilutions of antagonist compounds starting at a concentration of 200 µM in the polarization buffer. Samples were read after an incubation time of one hour. Fluorescence polarization values were plotted as a function of the antagonist concentration, and the IC₅₀ values were obtained by fitting the data to a 4-parameter equation using Kaleidagraph software (Synergy software, Reading, PA). *K*ᵢ values for the antagonists were determined from the IC₅₀ values according to the procedure of Keating, S.M., Marsters, J, Beresini, M., Ladner, C., Zioncheck, K., Clark, K., Arellano, F., and Bodary., S.(2000) in Proceedings of SPIE : In Vitro Diagnostic Instrumentation (Cohn, G.E., Ed.) pp 128-137, Bellingham, WA. Compounds of the invention that were tested in this assay exhibited IC₅₀ and *K*ᵢ values for the IAP BIR domain as shown in the table below. All values are micromolar. Compounds 8, 9, 12, 13, 15, 17 and 18 are compounds of the invention.

| Compd | C-IAP1 BIR3 | | C-IAP2 BIR3 | | MLXBIR3SG | | X-IAP BIR3 | |
|---|---|---|---|---|---|---|---|---|
| | IC₅₀ | Kᵢ | IC₅₀ | Kᵢ | IC₅₀ | Kᵢ | IC₅₀ | Kᵢ |
| 1 | 0.283 | 0.044 | 0.468 | 0.101 | 0.189 | 0.043 | 9.940 | 1.7690 |
| | 0.165 | 0.026 | 0.225 | 0.049 | 0.181 | 0.041 | 14.778 | 2.630 |
| 2 | 0.192 | 0.030 | 0.256 | 0.055 | 0.279 | 0.064 | 11.140 | 1.983 |
| 3 | 0.245 | 0.0380 | 0.272 | 0.059 | 0.178 | 0.041 | 11.151 | 1.985 |
| 5 | 2.118 | 0.328 | 3.476 | 0.751 | 4.822 | 1.099 | 13.550 | 2.412 |
| 6 | 0.131 | 0.020 | 0.222 | 0.048 | 0.248 | 0.057 | 8.836 | 1.573 |
| 7 | 14.209 | 3.123 | 26.976 | 4.568 | >200 | >46 | >200 | >35 |
| 8 | 3.120 | 0.483 | 1.941 | 0.419 | 0.899 | 0.205 | >200 | >35 |
| 9 | 5.844 | 0.905 | 20.572 | 4.445 | 5.740 | 1.308 | >200 | >35 |
| | 3.180 | 0.492 | 5.995 | 1.295 | 2.701 | 0.616 | >200 | >35 |
| 10 | 0.700 | 0.108 | 1.130 | 0.244 | 2.399 | 0.547 | 36.214 | 6.445 |
| 11 | 13.519 | 2.093 | 34.454 | 7.444 | ~60 | 13.675 | >200 | >35 |
| 12 | 6.877 | 1.065 | 19.131 | 4.133 | 2.541 | 0.579 | >200 | >35 |
| 13 | 3.885 | 0.602 | 6.246 | 1.349 | 4.225 | 0.963 | >200 | >35 |
| 14 | 0.1400 | 0.0217 | 0.2257 | 0.0488 | 0.250 | 0.0570 | 26.384 | 4.696 |
| 15 | 3.642 | 0.564 | 7.568 | 1.635 | 2.899 | 0.661 | >200 | >35 |
| 16 | 0.738 | 0.114 | 1.322 | 0.286 | 4.537 | 1.034 | >200 | >35 |
| 17 | 6.412 | 0.993 | 6.531 | 1.411 | 5.161 | 1.176 | >200 | >35 |
| 18 | 2.551 | 0.395 | 4.338 | 0.937 | 2.476 | 0.564 | >200 | >35 |
| 19 | 0.289 | 0.045 | 0.341 | 0.074 | 0.543 | 0.124 | ~100 | 17.797 |
| 20 | 0.133 | 0.021 | 0.202 | 0.044 | 0.249 | 0.057 | 21.732 | 3.868 |
| 21 | 0.202 | 0.031 | 0.342 | 0.074 | 0.407 | 0.093 | 22.411 | 3.989 |
| 22 | 2.287 | 0.354 | 2.454 | 0.530 | 8.881 | 2.024 | >200 | >35 |
| 23 | 0.155 | 0.024 | 0.276 | 0.060 | 0.304 | 0.069 | 13.977 | 2.487 |
| 24 | 1.325 | 0.205 | 1.319 | 0.285 | 4.076 | 0.929 | >200 | >35 |

## Claims

1. A compound having the formula (I): 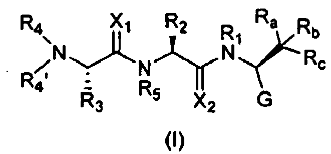 wherein:
Rₐ, R_{b} and R_{c} are each independently hydroxyl, halogen, alkyl, alkoxy, alkylthio or sulfonyl; wherein said alkyl, alkoxy, alkylthio and sulfonyl groups are optionally substituted with amido, carbamoyl and aryl which are optionally substituted with hydroxyl halogen and alkoxy; or two of Rₐ, R_{b} and R_{c} together form a carbocycle or heterocycle and the other of Rₐ, R_{b} and R_{c} is H, hydroxyl, halogen, alkyl, alkoxy, alkylthio or sulfonyl; or
Rₐ is H while R_{b} and R_{c} are each independently hydroxyl, halogen, alkyl, alkoxy, alkylthio or sulfonyl; wherein said alkyl, alkoxy, alkylthio and sulfonyl groups are optionally substituted with amido, carbamoyl and aryl which are optionally substituted with hydroxyl halogen and alkoxy; or two of Rₐ, R_{b} and R_{c} together form a carbocycle or heterocycle and the other of Rₐ, R_{b} and R_{c} is H, hydroxyl, halogen, alkyl, alkoxy, alkylthio or sulfonyl;
X₁ and X₂ are each independently O or S;
R₁ is H or alkyl;
R₂ is alkyl, a carbocycle, carbocyclylalkyl, a heterocycle or heterocyclylalkyl each optionally substituted with halogen, hydroxyl, oxo, thione, mercapto, carboxyl, alkyl, haloalkyl, alkoxy, alkylthio, sulfonyl, amino and nitro;
R₃ is H or alkyl optionally substituted with halogen or hydroxyl; or R₃ and R₄ together form a 3-6 heterocycle;
R₄ and R₄' are independently H, hydroxyl, amino, alkyl, carbocycle, carbocycloalkyl, carbocycloalkyloxy, carbocycloalkyloxycarbonyl, heterocycle, heterocycloalkyl, heterocycloalkyloxy or heterocycloalkyloxycarbonyl; wherein each alkyl, carbocycloalkyl, carbocydoalkyloxy, carbocycloalkyloxycarbonyl, heterocycle, heterocycloalkyl, heterocycloalkyloxy and heterocycloalkyloxycarbonyl is optionally substituted with halogen, hydroxyl, mercapto, carboxyl, alkyl, alkoxy, amino, imino and nitro; or R₄ and R₄' together form a heterocycle;
R₅ is H or alkyl;
G is: 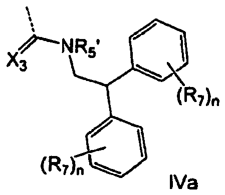 wherein:
R₅' is H or alkyl;
R₇ in each occurrence is independently H, cyano, hydroxyl, mercapto, halogen, nitro, carboxyl, amidino, guanidino, alkyl, a carbocycle, a heterocycle or -U-V; wherein U is -O-, -S-, -S(O)-, S(O)₂, -N(R₈)-, -C(O)-, -C(O)-NR₈-, -NR₈-C(O)-, -SO₂-NR₈-, -NR₈-SO₂-, -NR₈-C(O)-NR₈-, -NR₉-C(NH)-NR₈-, -NR₈-C(NH)-, -C(O)-O- or -O-C(O)- and V is alkyl, a carbocycle or a heterocycle; and wherein one or more CH₂ or CH groups of an alkyl is optionally replaced with -O-, -S-, -S(O)-, S(O)₂, -N(R₈)-, -C(O)-, -C(O)-NR₈-, -NR₈-C(O)-, -SO₂-NR₈-, -NR₈-SO₂-, -NR₈-C(O)-NR₈-, -C(O)-O- or -O-C(O)-; and an alkyl, carbocycle and heterocycle is optionally substituted with hydroxyl, alkoxy, acyl, halogen, mercapto, oxo, carboxyl, acyl, halo-substituted alkyl, amino, cyano nitro, amidino, guanidino an optionally substituted carbocycle or an optionally substituted heterocycle;
R₈ is H, alkyl, a carbocycle or a heterocycle wherein one or more CH₂ or CH groups of said alkyl is optionally replaced with -O-, -S-, -S(O)-, S(O)₂, -N(R₈), or -C(O)-; and said alkyl, carbocycle and heterocycle is optionally substituted with hydroxyl, alkoxy, acyl, halogen, mercapto, oxo (=O), carboxyl, acyl, halo-substituted alkyl, amino, cyano nitro, amidino, guanidino an optionally substituted carbocycle or an optionally substituted heterocycle;
X₃ is O or S; and
n in each occurrence is 1 to 4.

2. A compound according to claim 1, wherein R₁ is H.

3. A compound according to claim 1 or 2, wherein R₂ is alkyl, cycloalkyl or a heterocycle.

4. A compound according to claim 1 or 2, wherein R₂ is a carbocycle or a heterocycle.

5. A compound according to claim 1 or 2, wherein R₂ is selected from t-butyl, isopropyl, cyclohexyl, tetrahydropyran-4-yl, N-methylsulfonylpiperidin-4-yl, tetrahydrothiopyran-4-yl, tetrahydrothiopyran-4-yl (in which the S is in oxidized form SO or SO₂), cyclohexan-4-one, 4-hydroxycyclohexane, 4-hydroxy-4-methylcyclohexane, 1-methyl-tetrahydropyran-4-yl, 2-hydroxyprop-2-yl, but-2-yl, thiophen-3-yl, piperidin-4-yl, N-acetylpiperidin-4-yl, N-hydroxyethylpiperidin-4-yl, N-(2-hydroxyacetyl)piperidin-4-yl, N-(2-methoxyacetyl)piperidin-4-yl, pyridin-3-yl, phenyl and 1-hydoxyeth-1-yl.

6. A compound according to any one of claims 1 to 5, wherein R₃ is methyl.

7. A compound according to any one of claims 1 to 6, wherein R₄ is H or methyl, and R₄' is H.

8. A compound according to any one of claims 1 to 7, wherein R₅ is H or methyl.

9. A compound according to any one of claims 1 to 8, wherein X₁ and X₂ are both O.

10. A pharmaceutical composition comprising a compounds according to any one of claims 1 to 9 and a therapeutically inert carrier, diluent or excipient.

11. A compound according to any one of claims 1 to 9 for use in the treatment of the human or animal body by therapy.

12. A compound according to any one of claims 1 to 9 for use in the treatment of a disease or condition associated with the overexpression of an IAP in a mammal.

13. A compound according to any one of claims 1 to 9 for use in the treatment of cancer.

14. Use of a compound according to any one of claims 1 to 9 in the manufacture of a medicament for use in the treatment of a disease or condition associated with the overexpression of an IAP in a mammal.

15. Use of a compound according to any one of claims 1 to 9 in the manufacture of a medicament for use in the treatment of cancer.

## Patentansprüche

1. Verbindung der Formel (I): 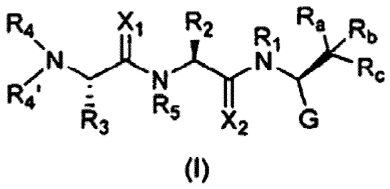 worin:
Rₐ, R_{b} und R_{c} jeweils unabhängig voneinander Hydroxyl, Halogen, Alkyl, Alkoxy, Alkylthio oder Sulfonyl sind; worin die Alkyl-, Alkoxy-, Alkylthio- oder Sulfonyl-gruppen gegebenenfalls mit Amido, Carbamoyl und Aryl substituiert sind, die gegebenenfalls mit Hydroxylhalogen und Alkoxy substituiert sind; oder zwei von Rₐ, R_{b} und R_{c} zusammen ein Carbocyclyl oder Heterocyclyl bilden und das jeweils andere von Rₐ, R_{b} und R_{c} H, Hydroxyl, Halogen, Alkyl, Alkoxy, Alkylthio oder Sulfonyl ist; oder
Rₐ = H ist, während R_{b} und R_{c} jeweils unabhängig voneinander Hydroxyl, Halogen, Alkyl, Alkoxy, Alkylthio oder Sulfonyl sind; worin die Alkyl-, Alkoxy-, Alkylthio- oder Sulfonyl-gruppen gegebenenfalls mit Amido, Carbamoyl und Aryl substituiert sind, die gegebenenfalls mit Hydroxylhalogen und Alkoxy substituiert sind; oder zwei von Rₐ, R_{b} und R_{c} zusammen ein Carbocyclyl oder Heterocyclyl bilden und das jeweils andere von Rₐ, R_{b} und R_{c} = H, Hydroxyl, Halogen, Alkyl, Alkoxy, Alkylthio oder Sulfonyl ist;
X₁ und X₂ jeweils unabhängig voneinander O oder S sind;
R₁ = H oder Alkyl ist;
R₂ Alkyl, ein Carbocyclyl, Carbocyclylalkyl, ein Heterocyclyl oder Heterocyclyl-alkyl, jeweils gegebenenfalls mit Halogen, Hydroxyl, Oxo, Thion, Mercapto, Carboxyl, Alkyl, Halogenalkyl, Alkoxy, Alkylthio, Sulfonyl, Amino und Nitro substituiert, ist;
R₃ = H oder Alkyl ist, das gegebenenfalls mit Halogen oder Hydroxyl substituiert ist; oder R₃ und R₄ zusammen ein 3- bis 6-gliedriges Heterocyclyl bilden;
R₄ und R₄' jeweils unabhängig voneinander H, Hydroxyl, Amino, Alkyl, Carbocyclyl, Carbocycloalkyl, Carbocycloalkoxy, Carbocycloalkyloxycarbonyl, Heterocyclyl, Heterocycloalkyl, Heterocycloalkoxy oder Heterocycloalkyloxycarbonyl sind; worin jeder Rest Alkyl, Carbocyclyl, Carbocycloalkyl, Carbocycloalkoxy, Carbocycloalkyloxy-carbonyl, Heterocyclyl, Heterocycloalkyl, Heterocycloalkoxy und Heterocycloalkyloxycarbonyl gegebenenfalls mit Halogen, Hydroxyl, Mercapto, Carboxyl, Alkyl, Alkoxy, Amino, Imino und Nitro substituiert ist; oder R₄ und R₄' zusammen ein Heterocyclyl bilden;
R₅ = H oder Alkyl ist;
G Folgendes ist: 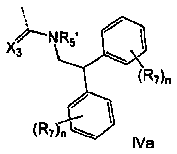
worin:
R₅' = H oder Alkyl ist;
R₇ bei jedem Auftreten jeweils H, Cyano, Hydroxyl, Mercapto, Halogen, Nitro, Carboxyl, Amidino, Guanidino, Alkyl, ein Carbocyclyl, ein Heterocyclyl oder -U-V ist; worin U = -O-, -S-, -S(O)-, -S(O)₂, -N(R₈)-, -C(O)-, -C(O)-NR₈-, -NR₈-C(O)-, -SO₂-NR₈-, NR₈-SO₂-, NR₈-C(O)-NR₈-, -NR₈-C(NH)-NR₈-, -NR₈-C(NH)-, -C(O)-O-oder -O-C(O)- ist und V Alkyl, ein Carbocyclyl oder ein Heterocyclyl ist; und worin eine oder mehrere CH₂- oder CH-Gruppen eines Alkylrests gegebenenfalls durch -O-, -S-, -S(O)-, -S(O)₂, -N(R₈)-, -C(O)-, -C(O)-NR₈-, -NR₈-C(O)-, -SO₂-NR₈-, NR₈-SO₂-, NR₈-C(O)-NR₈-, -C(O)-O- oder -O-C(O)- ersetzt sind; und ein Alkyl, ein Carbocyclyl und ein Heterocyclyl gegebenenfalls mit Hydroxyl, Alkoxy, Acyl, Halogen, Mercapto, Oxo, Carboxyl, Acyl, halogensubstituiertem Alkyl, Amino, Cyano, Nitro, Amidino, Guanidino, einem gegebenenfalls substituierten Carbocyclyl oder einem gegebenenfalls substituierten Heterocyclyl substituiert sind;
R₈ = H, ein Carbocyclyl oder ein Heterocyclyl ist, worin eine oder mehrere CH₂- oder CH-Gruppen des Alkylrests gegebenenfalls durch -O-, -S-, -S(O)-, -S(O)₂, -N(R₈) oder -C(O)- ersetzt sind; und das Alkyl, das Carbocyclyl und das Heterocyclyl gegebenenfalls mit Hydroxyl, Alkoxy, Acyl, Halogen, Mercapto, Oxo (=O), Carboxyl, Acyl, halogensubstituiertem Alkyl, Amino, Cyano, Nitro, Amidino, Guanidino, einem gegebenenfalls substituierten Carbocyclyl oder einem gegebenenfalls substituierten Heterocyclyl substituiert sind;
X₃ = O oder S ist; und
n bei jedem Auftreten 1 bis 4 ist.

2. Verbindung nach Anspruch 1, worin R₁ = H ist.

3. Verbindung nach Anspruch 1 oder 2, worin R₂ Alkyl, Cycloalkyl oder ein Heterocyclyl ist.

4. Verbindung nach Anspruch 1 oder 2, worin R₂ ein Carbocyclyl oder ein Heterocyclyl ist.

5. Verbindung nach Anspruch 1 oder 2, worin R₂ aus t-Butyl, Isopropyl, Cyclohexyl, Tetrahydropyran-4-yl, N-Methylsulfonylpiperidin-4-yl, Tetrahydrothiopyran-4-yl, Tetrahydrothiopyran-4-yl (wobei der S in oxidierter Form als SO oder SO₂ vorliegt), Cyclohexan-4-on, 4-Hydroxycyclohexan, 4-Hydroxy-4-methylcyclohexan, 1-Methyl-tetrahydropyran-4-yl, 2-Hydroxyprop-2-yl, But-2-yl, Thiophen-3-yl, Piperidin-4-yl, N-Acetylpiperidin-4-yl, N-Hydroxyethylpiperidin-4-yl, N-(2-Hydroxyacetyl)piperidin-4-yl, N-(2-Methoxyacetyl)piperidin-4-yl, Pyridin-3-yl, Phenyl und 1-Hydroxyeth-1-yl ausgewählt ist.

6. Verbindung nach einem der Ansprüche 1 bis 5, worin R₃ Methyl ist.

7. Verbindung nach einem der Ansprüche 1 bis 6, worin R₄ = H oder Methyl ist und R₄' = H ist.

8. Verbindung nach einem der Ansprüche 1 bis 7, worin R₅ = H oder Methyl ist.

9. Verbindung nach einem der Ansprüche 1 bis 8, worin X₁ und X₂ beide O sind.

10. Pharmazeutische Zusammensetzung, die eine Verbindung nach einem der Ansprüche 1 bis 9 und einen therapeutisch inerten Träger, Verdünner oder Exzipienten umfasst.

11. Verbindung nach einem der Ansprüche 1 bis 9 zur Verwendung bei der Behandlung des menschlichen oder tierischen Körpers durch Therapie.

12. Verbindung nach einem der Ansprüche 1 bis 9 zur Verwendung bei der Behandlung einer Krankheit oder eines Leidens im Zusammenhang mit der Überexpression eines IAP bei einem Säugetier.

13. Verbindung nach einem der Ansprüche 1 bis 9 zur Verwendung bei der Behandlung von Krebs.

14. Verwendung einer Verbindung nach einem der Ansprüche 1 bis 9 bei der Herstellung eines Medikaments zur Verwendung bei der Behandlung einer Krankheit oder eines Leidens im Zusammenhang mit der Überexpression eines IAP bei einem Säugetier.

15. Verwendung einer Verbindung nach einem der Ansprüche 1 bis 9 bei der Herstellung eines Medikaments zur Verwendung bei der Behandlung von Krebs.

## Revendications

1. Composé répondant à la formule (I) : 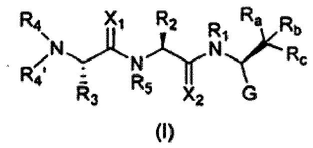 dans laquelle :
Rₐ, R_{b} et R_{c} représentent chacun indépendamment un groupe hydroxyle, halogéno, alkyle, alkoxy, alkylthio ou sulfonyle ; lesdits groupes alkyle, alkoxy, alkylthio et sulfonyle étant facultativement substitués avec des groupes amido, carbamoyle et aryle qui sont facultativement substitués avec des substituants hydroxyle, halogéno et alkoxy ; ou bien deux de Rₐ, R_{b} et R_{c} forment conjointement un carbocycle ou hétérocycle et l'autre de Rₐ, R_{b} et R_{c} représente H, un groupe hydroxyle, halogéno, alkyle, alkoxy, alkylthio ou sulfonyle ; ou bien
Rₐ représente H tandis que R_{b} et R_{c} représentent chacun indépendamment un groupe hydroxyle, halogéno, alkyle, alkoxy, alkylthio ou sulfonyle ; lesdits groupes alkyle, alkoxy, alkylthio et sulfonyle étant facultativement substitués avec des substituants amido, carbamoyle et aryle qui sont facultativement substitués avec des substituants hydroxyle, halogéno et alkoxy ; ou bien deux de Rₐ, R_{b} et R_{c} forment conjointement un carbocycle ou hétérocycle et l'autre de Rₐ, R_{b} et R_{c} représente H, un groupe hydroxyle, halogéno, alkyle, alkoxy, alkylthio ou sulfonyle ;
X₁ et X₂ représentent chacun indépendamment O ou S ;
R₁ représente H ou un groupe alkyle ;
R₂ représente un groupe alkyle, un carbocycle, un groupe carbocyclylalkyle, un hétérocycle ou un groupe hétérocyclylalkyle, chacun facultativement substitué avec des substituants halogéno, hydroxyle, oxo, thione, mercapto, carboxyle, alkyle, halogénalkyle, alkoxy, alkylthio, sulfonyle, amino et nitro ;
R₃ représente H ou un groupe alkyle facultativement substitué avec un substituant halogéno ou hydroxyle ; ou bien R₃ et R₄ forment conjointement un hétérocycle tri- à hexagonal ;
R₄ et R₄' représentent indépendamment H, un groupe hydroxyle, amino, alkyle, carbocyclique, carbocycloalkyle, carbocycloalkyloxy, carbocycloalkyloxycarbonyle, hétérocyclique, hétérocycloalkyle, hétérocycloalkyloxy ou hétérocycloalkyl-oxycarbonyle ; chacun des groupes alkyle, carbocycloalkyle, carbocycloalkyloxy, carbocycloalkyloxycarbonyle, hétérocyclique, hétérocycloalkyle, hétérocycloalkyloxy et hétérocycloalkyl-oxycarbonyle étant facultativement substitué avec des substituants halogéno, hydroxyle, mercapto, carboxyle, alkyle, alkoxy, amino, imino et nitro ; ou bien R₄ et R₄' forment conjointement un hétérocycle ;
R₅ représente H ou un groupe alkyle;
G représente un groupe : 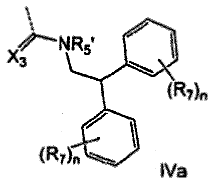 dans lequel:
R₅' représente H ou un groupe alkyle;
R₇, à chaque occurrence représente indépendamment H, un groupe cyano, hydroxyle, mercapto, halogéno, nitro, carboxyle, amidino, guanidino, alkyle, un carbocycle, un hétérocycle ou un groupe -U-V ; U représentant un groupe -O-, -S-, -S(O)-, S(O)₂, -N(R₈)-, -C(O)-, -C(O)-, -C(O)-NR₈-, -NR₈-C(O)-, -SO₂-NR₈-, -NR₈-SO₂-, -NR₈-C(O)NR₈-, -NR₈-C(NH)-NR₈-, -NR₈-C(NH)-, -C(O)-O- ou -O-C(O)- et V représentant un groupe alkyle, un carbocycle ou un hétérocycle ; et un ou plusieurs groupes CH₂ ou CH d'un groupe alkyle étant facultativement remplacés par un groupe -O-, -S-, -S(O)-, S(O)₂, -N(R₈)-, -C(O), -C(O)-NR₈-, -NR₈-C(O)-, -SO₂-NR₈-, -NR₈-SO₂-, -NR₈-C(O)-NR₈-, -C(O)O- ou -O-C(O)- ; et un groupe alkyle, un carbocycle ou un hétérocycle étant facultativement substitué avec des substituants hydroxyle, alkoxy, acyle, halogéno, mercapto, oxo, carboxyle, acyle, alkyle à substituant halogéno, amino, cyano, nitro, amidino, guanidino, un carbocycle facultativement substitué ou un hétérocycle facultativement substitué ;
R₈ représente H, un groupe alkyle, un carbocycle ou un hétérocycle, dans lequel ou plusieurs groupes CH₂ ou CH dudit groupe alkyle sont facultativement remplacés par un groupe -O-, -S-, -S(O)-, S(O)₂, -N(R₈)- ou -C(O) ; et ledit groupe alkyle, ledit carbocycle et ledit hétérocycle sont facultativement substitués avec des substituants hydroxyle, alkoxy, acyle, halogéno, mercapto, oxo(=O), carboxyle, acyle, alkyle à substituant halogéno, amino, cyano, nitro, amidino, guanidino, un carbocycle facultativement substitué ou un hétérocycle facultativement substitué ;
X₃ représente O ou S ; et
n, à chaque occurrence, a une valeur de 1 à 4.

2. Composé suivant la revendication 1, dans lequel R₁ représente H.

3. Composé suivant la revendication 1 ou 2, dans lequel R₂ représente un groupe alkyle, cycloalkyle ou un hétérocycle.

4. Composé suivant la revendication 1 ou 2, dans lequel R₂ représente un carbocycle ou un hétérocycle.

5. Composé suivant la revendication 1 ou 2, dans lequel R₂ est choisi entre des groupes tertiobutyle, isopropyle, cyclohexyle, tétrahydropyranne-4-yle, N-méthylsulfonyl-pipéridine-4-yle, tétrahydrothiopyranne-4-yle, tétrahydro-thiopyranne-4-yle (dans lequel l'atome de S est sous la forme oxydée SO ou SO₂), cyclohexane-4-one, 4-hydroxy-cyclohexane, 4-hydroxy-4-méthylcyclohexane, 1-méthyl-tétra-hydropyranne-4-yle, 2-hydroxyprop-2-yle, but-2-yle, thiophène-3-yle, pipéridine-4-yle, N-acétylpipéridine-4-yle, N-hydroxy-éthylpipéridine-4-yle, N-(2-hydroxyacétyl)pipéridine-4-yle, N-(2-méthoxyacétyl)pipéridine-4-yle, pyridine-3-yle, phényle et 1-hydroxyéth-1-yle.

6. Composé suivant l'une quelconque des revendications 1 à 5, dans lequel R₃ représente un groupe méthyle.

7. Composé suivant l'une quelconque des revendications 1 à 6, dans lequel R₄ représente H ou un groupe méthyle et R₄' représente H.

8. Composé suivant l'une quelconque des revendications 1 à 7, dans lequel R₅ représente H ou un groupe méthyle.

9. Composé suivant l'une quelconque des revendications 1 à 8, dans lequel X₁ et X₂ représentent tous deux O.

10. Composition pharmaceutique comprenant un composé suivant l'une quelconque des revendications 1 à 9 et un support, diluant ou excipient thérapeutiquement inerte.

11. Composé suivant l'une quelconque des revendications 1 à 9, pour une utilisation dans le traitement de l'organisme humain ou animal par thérapie.

12. Composé suivant l'une quelconque des revendications 1 à 9, pour une utilisation dans le traitement d'une maladie ou affection associée à la surexpression d'une IAP chez un mammifère.

13. Composé suivant l'une quelconque des revendications 1 à 9, pour une utilisation dans le traitement du cancer.

14. Utilisation d'un composé suivant l'une quelconque des revendications 1 à 9, dans la production d'un médicament pour une utilisation dans le traitement d'une maladie ou affection associée à la surexpression d'une IAP chez un mammifère.

15. Utilisation d'un composé suivant l'une quelconque des revendications 1 à 9, dans la production d'un médicament pour une utilisation pour une utilisation dans le traitement du cancer.
